# EUROPEAN PATENT APPLICATION

(11) **EP 3 088 535 A1**
(43) Date of publication of application: **02.11.2016**
(21) Application number: 15001269.8
(22) Date of filing: 29.04.2015
(51) Int. Cl.: C12Q 1/70

(54) **METHODS FOR IDENTIFYING ANTIRETROVIRAL COMPOUNDS TARGETING THE REVERSE TRANSCRIPTASE OF HIV**

(71) Applicant: Amikana. Biologics Faculté de Médecine de la Timone, 13005 Marseille (FR)
(72) Inventor: Gluschankof, Pablo, 13001 Marseille (FR); Charifi, Mohammed-Ferose, 13006 Marseille (FR)
(74) Representative: Barbot, Willy

(57) **Abstract**

The invention relates to a method for identifying a compound inhibiting HIV reverse transcriptase (HIV RT), said method comprising the steps of :
a) Transforming a yeast cell by at least one nucleic acid encoding for a HIV RT and a signal peptide, wherein the nucleic acid sequence encoding for HIV RT is under the control of a promoter and the signal peptide enables the secretion of the HIV RT in the supernatant;
b) Culturing the transformed yeast cell of step a) for secretion of the HIV RT in the supernatant;
c) Separating the cultured transformed yeast cells of step b) from the supernatant for collecting said supernatant containing the HIV RT;
d) Assaying the activity of said HIV RT in the collected supernatant of step c) in the presence or absence of a compound; and
e) Selecting a compound inhibiting HIV RT activity in step d).

The invention further provides kits for carrying out the method of the invention.

SEQUENCE LISTING

<110>  AMIKANA Biologics
       GLUSCHANKOF, Pablo
       CHARIFI, Mohammed-Ferose

<120>  METHODS FOR IDENTIFYING ANTIRETROVIRAL COMPOUNDS TARGETING THE
       REVERSE TRANSCRIPTASE OF HIV

<130>  AMI-B-0006-EP1

<160>  32

<170>  PatentIn version 3.5

<210>  1
<211>  330
<212>  DNA
<213>  Artificial Sequence

<220>
<223>  Prepro-MF  signal peptide

<400>  1
atgagatttc cttcaatttt tactgcagtt ttattcgcag catcctccgc attagctgct      60

ccagtcaaca ctacaacaga agatgaaacg gcacaaattc cggctgaagc tgtcatcggt     120

tacttagatt tagaagggga tttcgatgtt gctgttttgc cattttccaa cagcacaaat     180

aacgggttat tgtttataaa tactactatt gccagcattg ctgctaaaga agaaggggta     240

tctttggata aaagagaggc tgaagcttgg cattggttgc aactaaaacc tggccaacca     300

atgtacaaga gagaagccga agctgaagct                                      330


<210>  2
<211>  267
<212>  DNA
<213>  Artificial Sequence

<220>
<223>  Prepro signal peptide

<400>  2
atgagatttc cttcaatttt tactgcagtt ttattcgcag catcctccgc attagctgct      60

ccagtcaaca ctacaacaga agatgaaacg gcacaaattc cggctgaagc tgtcatcggt     120

tacttagatt tagaagggga tttcgatgtt gctgttttgc cattttccaa cagcacaaat     180

aacgggttat tgtttataaa tactactatt gccagcattg ctgctaaaga agaaggggta     240

tctttggata aaagagaggc tgaagct                                         267


<210>  3
<211>  147
<212>  DNA
<213>  Artificial Sequence

<220>

<223>  K28 signal peptide

<400>  3
atggagagcg tttcctcatt atttaacatt ttttcaacaa tcatggttaa ctataaatcg      60

ttagttctag cactattaag tgtttcaaat ctcaaatatg cacggggtat gccgacatct      120

gagagacagc agggcttaga agaacgt                                          147


<210>  4
<211>  20
<212>  DNA
<213>  Artificial Sequence

<220>
<223>  PRO1 primer

<400>  4
cagagccaac agccccacca                                                   20


<210>  5
<211>  22
<212>  DNA
<213>  Artificial Sequence

<220>
<223>  RT20 primer

<400>  5
ctgccagttc tagctctgct tc                                                22


<210>  6
<211>  50
<212>  DNA
<213>  Artificial Sequence

<220>
<223>  HIV1-RT 5' primer

<400>  6
caagagagaa gccgaagctg aagctcccat tagccctatt gagactgtac                  50


<210>  7
<211>  31
<212>  DNA
<213>  Artificial Sequence

<220>
<223>  HIV1-RT 3' primer

<400>  7
taaggagttt acataattgc cttactttaa t                                      31


<210>  8
<211>  1681
<212>  DNA
<213>  Human immunodeficiency virus type 1

<400> 8
cccattagcc ctattgagac tgtaccagta aaattaaagc caggaatgga tggcccaaaa 60

gttaaacaat ggccattgac agaagaaaaa ataaaagcat tagtagaaat ttgtacagag 120

atggaaaagg aagggaaaat ttcaaaaatt gggcctgaaa atccatacaa tactccagta 180

tttgccataa agaaaaaaga cagtactaaa tggagaaaat tagtagattt cagagaactt 240

aataagagaa ctcaagactt ctgggaagtt caattaggaa taccacatcc cgcagggtta 300

aaaaagaaaa aatcagtaac agtactggat gtgggtgatg catatttttc agttccctta 360

gatgaagact tcaggaagta tactgcattt accataccta gtataaacaa tgagacacca 420

gggattagat atcagtacaa tgtgcttcca cagggatgga aaggatcacc agcaatattc 480

caaagtagca tgacaaaaat cttagagcct tttagaaaac aaaatccaga catagttatc 540

tatcaataca tggatgattt gtatgtagga tctgacttag aaatagggca gcatagaaca 600

aaaatagagg agctgagaca acatctgttg aggtggggac ttaccacacc agacaaaaaa 660

catcagaaag aacctccatt cctttggatg ggttatgaac tccatcctga taaatggaca 720

gtacagccta tagtgctgcc agaaaaagac agctggactg tcaatgacat acagaagtta 780

gtggggaaat tgaattgggc aagtcagatt tacccaggga ttaaagtaag gcaattatgt 840

aaactcctta gaggaaccaa agcactaaca gaagtaatac cactaacaga agaagcagag 900

ctagaactgg cagaaaacag agagattcta aaagaaccag tacatggagt gtattatgac 960

ccatcaaaag acttaatagc agaaatacag aagcaggggc aaggccaatg gacatatcaa 1020

atttatcaag agccatttaa aaatctgaaa acaggaaaat atgcaagaat gaggggtgcc 1080

cacactaatg atgtaaaaca attaacagag gcagtgcaaa aaataaccac agaaagcata 1140

gtaatatggg gaaagactcc taaatttaaa ctgcccatac aaaaggaaac atgggaaaca 1200

tggtggacag agtattggca agccacctgg attcctgagt gggagtttgt taatacccct 1260

cccttagtga aattatggta ccagttagag aaagaaccca tagtaggagc agaaaccttc 1320

tatgtagatg gggcagctaa cagggagact aaattaggaa aagcaggata tgttactaat 1380

agaggaagac aaaaagttgt caccctaact gacacaacaa atcagaagac tgagttacaa 1440

gcaatttatc tagctttgca ggattcggga ttagaagtaa acatagtaac agactcacaa 1500

tatgcattag gaatcattca agcacaacca gatcaaagtg aatcagagtt agtcaatcaa 1560

ataatagagc agttaataaa aaaggaaaag gtctatctgg catgggtacc agcacacaaa 1620

ggaattggag gaaatgaaca agtagataaa ttagtcagtg ctggaatcag gaaagtacta 1680

t 1681

<210> 9
<211> 60
<212> DNA

<213>  Artificial Sequence

<220>
<223>  FpcRH primer

<400>  9
tatactttaa cgtcaaggag aaaaaacccc ggatccatgt ggggtagaga caacaactcc      60


<210>  10
<211>  53
<212>  DNA
<213>  Artificial Sequence

<220>
<223>  R2139 primer

<400>  10
gagctcttaa tccattcctg gctttaattt tactggtaca gtttcaattg gac      53


<210>  11
<211>  52
<212>  DNA
<213>  Artificial Sequence

<220>
<223>  HIV1-PR 3' primer

<400>  11
tgagcttaat cagctcttaa tccattcctg gctttaattt tactggtaca gt      52


<210>  12
<211>  3012
<212>  DNA
<213>  Human immunodeficiency virus type 1

<400>  12
tttttagggg aagatctggc cttcctacaa gggaaggcca gggaattttc ttcagagcag      60

accagagcca acagccccac cagaagagag cttcaggtct ggggtagaga caacaactcc      120

ccctcagaag caggagccga tagacaagga actgtatcct ttaacttccc tcaggtcact      180

ctttggcaac gacccctcgt cacaataaag ataggggggc aactaaagga agctctatta      240

gatacaggag cagatgatac agtattagaa gaaatgagtt tgccaggaag atggaaacca      300

aaaatgatag ggggaattgg aggttttatc aaagtaagac agtatgatca gatactcata      360

gaaatctgtg gacataaagc tataggtaca gtattagtag gacctacacc tgtcaacata      420

attggaagaa atctgttgac tcagattggt tgcactttaa attttcccat tagccctatt      480

gagactgtac cagtaaaatt aaagccagga atggatggcc caaaagttaa acaatggcca      540

ttgacagaag aaaaaataaa agcattagta gaaatttgta cagagatgga aaaggaaggg      600

aaaatttcaa aaattgggcc tgaaaatcca tacaatactc cagtatttgc cataaagaaa      660

aaagacagta ctaaatggag aaaattagta gatttcagag aacttaataa gagaactcaa      720

gacttctggg aagttcaatt aggaatacca catcccgcag ggttaaaaaa gaaaaaatca 780

gtaacagtac tggatgtggg tgatgcatat ttttcagttc ccttagatga agacttcagg 840

aagtatactg catttaccat acctagtata aacaatgaga caccagggat tagatatcag 900

tacaatgtgc ttccacaggg atggaaagga tcaccagcaa tattccaaag tagcatgaca 960

aaaatcttag agccttttag aaaacaaaat ccagacatag ttatctatca atacatggat 1020

gatttgtatg taggatctga cttagaaata gggcagcata gaacaaaaat agaggagctg 1080

agacaacatc tgttgaggtg gggacttacc acaccagaca aaaaacatca gaaagaacct 1140

ccattccttt ggatgggtta tgaactccat cctgataaat ggacagtaca gcctatagtg 1200

ctgccagaaa aagacagctg gactgtcaat gacatacaga agttagtggg gaaattgaat 1260

tgggcaagtc agatttaccc agggattaaa gtaaggcaat tatgtaaact ccttagagga 1320

accaaagcac taacagaagt aataccacta acagaagaag cagagctaga actggcagaa 1380

aacagagaga ttctaaaaga accagtacat ggagtgtatt atgacccatc aaaagactta 1440

atagcagaaa tacagaagca ggggcaaggc caatggacat atcaaattta tcaagagcca 1500

tttaaaaatc tgaaaacagg aaaatatgca agaatgaggg gtgcccacac taatgatgta 1560

aaacaattaa cagaggcagt gcaaaaaata accacagaaa gcatagtaat atggggaaag 1620

actcctaaat ttaaactgcc catacaaaag gaaacatggg aaacatggtg gacagagtat 1680

tggcaagcca cctggattcc tgagtgggag tttgttaata cccctccctt agtgaaatta 1740

tggtaccagt tagagaaaga acccatagta ggagcagaaa ccttctatgt agatggggca 1800

gctaacaggg agactaaatt aggaaaagca ggatatgtta ctaatagagg aagacaaaaa 1860

gttgtcaccc taactgacac aacaaatcag aagactgagt tacaagcaat ttatctagct 1920

ttgcaggatt cgggattaga agtaaacata gtaacagact cacaatatgc attaggaatc 1980

attcaagcac aaccagatca aagtgaatca gagttagtca atcaaataat agagcagtta 2040

ataaaaaagg aaaaggtcta tctggcatgg gtaccagcac acaaaggaat tggaggaaat 2100

gaacaagtag ataaattagt cagtgctgga atcaggaaag tactattttt agatggaata 2160

gataaggccc aagatgaaca tgagaaatat cacagtaatt ggagagcaat ggctagtgat 2220

tttaacctgc cacctgtagt agcaaaagaa atagtagcca gctgtgataa atgtcagcta 2280

aaaggagaag ccatgcatgg acaagtagac tgtagtccag gaatatggca actagattgt 2340

acacatttag aaggaaaagt tatcctggta gcagttcatg tagccagtgg atatatagaa 2400

gcagaagtta ttccagcaga aacagggcag gaaacagcat attttctttt aaaattagca 2460

ggaagatggc cagtaaaaac aatacatact gacaatggca gcaatttcac cggtgctacg 2520

gttagggccg cctgttggtg ggcgggaatc aagcaggaat ttggaattcc ctacaatccc 2580

caaagtcaag gagtagtaga atctatgaat aaagaattaa agaaaattat aggacaggta 2640

agagatcagg ctgaacatct taagacagca gtacaaatgg cagtattcat ccacaatttt 2700

aaaagaaaag gggggattgg ggggtacagt gcaggggaaa gaatagtaga cataatagca 2760

acagacatac aaactaaaga attacaaaaa caaattacaa aaattcaaaa ttttcgggtt 2820

tattacaggg acagcagaaa tccactttgg aaaggaccag caaagctcct ctggaaaggt 2880

gaaggggcag tagtaataca agataatagt gacataaaag tagtgccaag aagaaaagca 2940

aagatcatta gggattatgg aaaacagatg gcaggtgatg attgtgtggc aagtagacag 3000

gatgaggatt ag 3012

<210> 13
<211> 295
<212> DNA
<213> Human immunodeficiency virus type 1

<400> 13
tcaggtcact ctttggcaac gacccctcgt cacaataaag ataggggggc aactaaagga 60

agctctatta gatacaggag cagatgatac agtattagaa gaaatgagtt tgccaggaag 120

atggaaacca aaaatgatag ggggaattgg aggttttatc aaagtaagac agtatgatca 180

gatactcata gaaatctgtg gacataaagc tataggtaca gtattagtag gacctacacc 240

tgtcaacata attggaagaa atctgttgac tcagattggt tgcactttaa atttt 295

<210> 14
<211> 1317
<212> DNA
<213> Human immunodeficiency virus type 2

<400> 14
ccagtcgcca agttagaccc aataaaagta acattgaagc cagggaaaga tggaccaagg 60

ctgaaacaat ggcccctaac aaaagaaaaa atagaagcac taaaagaaat ttgtgaaaaa 120

atggaaaggg agggccaact agaagaagca cctccaacta atccttataa tacccccaca 180

tttgcaatta agaaaaagga caagaacaaa tggagaatgc taatagattt tagagaacta 240

aacagggtga ctcaagattt cacagaaatt cagctaggaa ttccacaccc ggcaggatta 300

gccaaaaaga aaaggattac tgtactagat gtaggggatg cctacttttc cataccacta 360

catgaagatt ttaggcaata tactgcattt accctaccat cagtaaacaa tgcagagcca 420

gaaaaaagat atgtatataa ggtcttacca caaggatgga aaggatcacc agcaatcttt 480

caattcatga tgaggcaaat cttagaacct ttcagaaaag caaacccaga cgtcattctc 540

atccaataca tggatgatat cttaatagct agtgacagga cgggtttaga gcatgacaaa 600

gtagtcctgc aactaaaaga acttctgaat ggcctagggt tctctacccc agatgagaag 660

ttccaaaagg accctccgtt tcaatggatg ggctatgaat tgtggccaac taaatggaaa 720

ctgcagaaaa tacaattacc tcagaaagaa atatggacag tcaatgacat ccaaaaacta 780

gtaggagttt tgaactgggc ggcgcagatc tatccaggga taaaaaccaa gcatttatgt 840

aaattgatta gaggaaaaat gacactcaca gaggaagtac agtggacaga gttagcagag 900

gcagaactag aagaaaacaa aattatctta agtcaggaac aagagggatc ctactatcag 960

gaagaagaag aactagaagc aacagtcatc aaaagccaag acaatcagtg ggcatacaaa 1020

atacaccagg gagagagggt tctaaaagta ggaaagtatg cgaagataaa aaatactcat 1080

accaatgggg tcagactact agcacaagta gtccaaaaaa taggaaagga agcactggtc 1140

atttggggac gagtgccaaa atttcaccta ccggtagaga gagacacctg ggagcaatgg 1200

tgggataact actggcaagt aacatgggtc ccagagtggg acttcgtatc taccccacca 1260

ctggtcaggt tgacatttaa cttggtagga gatcctatac caggcacaga gaccttt 1317

<210> 15
<211> 907
<212> DNA
<213> Artificial Sequence

<220>
<223> Gal 1-10 promoter

<400> 15
gaattcgaca ggttatcagc aacaacacag tcatatccat tctcaattag ctctaccaca 60

gtgtgtgaac caatgtatcc agcaccacct gtaaccaaaa caattttaga agtactttca 120

ctttgtaact gagctgtcat ttatattgaa ttttcaaaaa ttcttacttt ttttttggat 180

ggacgcaaag aagtttaata atcatattac atggcattac caccatatac atatccatat 240

acatatccat atctaatctt acttatatgt tgtggaaatg taaagagccc cattatctta 300

gcctaaaaaa accttctctt tggaactttc agtaatacgc ttaactgctc attgctatat 360

tgaagtacgg attagaagcc gccgagcggg tgacagccct ccgaaggaag actctcctcc 420

gtgcgtcctc gtcttcaccg gtcgcgttcc tgaaacgcag atgtgcctcg cgccgcactg 480

ctccgaacaa taaagattct acaatactag cttttatggt tatgaagagg aaaaattggc 540

agtaacctgg ccccacaaac cttcaaatga acgaatcaaa ttaacaacca taggatgata 600

atgcgattag ttttttagcc ttatttctgg ggtaattaat cagcgaagcg atgatttttg 660

atctattaac agatatataa atgcaaaaac tgcataacca ctttaactaa tactttcaac 720

attttcggtt tgtattactt cttattcaaa tgtaataaaa gtatcaacaa aaaattgtta 780

atatacctct atactttaac gtcaaggaga aaaaactata atgactaaat ctcattcaga 840

agaagtgatt gtacctgagt tcaattctag cgcaaaggaa ttaccaagac cattggccga 900

aaagtgc 907

<210>  16
<211>  1047
<212>  DNA
<213>  Artificial Sequence

<220>
<223>  ADH1 promoter

<400>  16
atgtctatcc cagaaactca aaaaggtgtt atcttctacg aatcccacgg taagttggaa      60

tacaaagata ttccagttcc aaagccaaag gccaacgaat tgttgatcaa cgttaaatac     120

tctggtgtct gtcacactga cttgcacgct tggcacggtg actggccatt gccagttaag     180

ctaccattag tcggtggtca cgaaggtgcc ggtgtcgttg tcggcatggg tgaaaacgtt     240

aagggctgga agatcggtga ctacgccggt atcaaatggt tgaacggttc ttgtatggcc     300

tgtgaatact gtgaattggg taacgaatcc aactgtcctc acgctgactt gtctggttac     360

acccacgacg gttctttcca acaatacgct accgctgacg ctgttcaagc cgctcacatt     420

cctcaaggta ccgacttggc ccaagtcgcc cccatcttgt gtgctggtat caccgtctac     480

aaggctttga agtctgctaa cttgatggcc ggtcactggg ttgctatctc cggtgctgct     540

ggtggtctag gttctttggc tgttcaatac gccaaggcta tgggttacag agtcttgggt     600

attgacggtg gtgaaggtaa ggaagaatta ttcagatcca tcggtggtga agtcttcatt     660

gacttcacta aggaaaagga cattgtcggt gctgttctaa aggccactga cggtggtgct     720

cacggtgtca tcaacgtttc cgtttccgaa gccgctattg aagcttctac cagatacgtt     780

agagctaacg gtaccaccgt tttggtcggt atgccagctg gtgccaagtg ttgttctgat     840

gtcttcaacc aagtcgtcaa gtccatctct attgttggtt cttacgtcgg taacagagct     900

gacaccagag aagctttgga cttcttcgcc agaggtttgg tcaagtctcc aatcaaggtt     960

gtcggcttgt ctaccttgcc agaaatttac gaaaagatgg aaaagggtca aatcgttggt    1020

agatacgttg ttgacacttc taaataa                                        1047


<210>  17
<211>  673
<212>  DNA
<213>  Artificial Sequence

<220>
<223>  GPD promoter

<400>  17
gagctcagtt tatcattatc aatactcgcc atttcaaaga atacgtaaat aattaatagt      60

agtgattttc ctaactttat ttagtcaaaa aattagcctt ttaattctgc tgtaacccgt     120

acatgcccaa aatagggggc gggttacaca gaatatataa catcgtaggt gtctgggtga     180

acagtttatt cctggcatcc actaaatata atggagcccg ctttttaagc tggcatccag     240

aaaaaaaaag aatcccagca ccaaaatatt gttttcttca ccaaccatca gttcataggt 300

ccattctctt agcgcaacta cagagaacag gggcacaaac aggcaaaaaa cgggcacaac 360

ctcaatggag tgatgcaacc tgcctggagt aaatgatgac acaaggcaat tgacccacgc 420

atgtatctat ctcattttct tacaccttct attaccttct gctctctctg atttggaaaa 480

agctgaaaaa aaaggttgaa accagttccc tgaaattatt cccctacttg actaataagt 540

atataaagac ggtaggtatt gattgtaatt ctgtaaatct atttcttaaa cttcttaaat 600

tctactttta tagttagtct tttttttagt tttaaaacac cagaacttag tttcgacgga 660

ttctagaact agt 673

<210> 18
<211> 675
<212> DNA
<213> Saccharomyces cerevisiae

<400> 18
atgtctgtta ttaatttcac aggtagttct ggtccattgg tgaaagtttg cggcttgcag 60

agcacagagg ccgcagaatg tgctctagat tccgatgctg acttgctggg tattatatgt 120

gtgcccaata gaaagagaac aattgacccg gttattgcaa ggaaaatttc aagtcttgta 180

aaagcatata aaaatagttc aggcactccg aaatacttgg ttggcgtgtt tcgtaatcaa 240

cctaaggagg atgttttggc tctggtcaat gattacggca ttgatatcgt ccaactgcat 300

ggagatgagt cgtggcaaga ataccaagag ttcctcggtt tgccagttat taaaagactc 360

gtatttccaa aagactgcaa catactactc agtgcagctt cacagaaacc tcattcgttt 420

attcccttgt ttgattcaga agcaggtggg acaggtgaac ttttggattg gaactcgatt 480

tctgactggg ttggaaggca agagagcccc gaaagcttac attttatgtt agctggtgga 540

ctgacgccag aaaatgttgg tgatgcgctt agattaaatg gcgttattgg tgttgatgta 600

agcggaggtg tggagacaaa tggtgtaaaa gactctaaca aaatagcaaa tttcgtcaaa 660

aatgctaaga aatag 675

<210> 19
<211> 17
<212> DNA
<213> Artificial Sequence

<220>
<223> RT5 primer

<400> 19
ctcctcgtac gggcgac 17

<210> 20
<211> 3569
<212> DNA

<213>  Artificial Sequence

<220>
<223>  Enterobacteriophage MS 2

<400>  20
tgggtggtaa ctagccaagc agctagttac caaatcggga gaatcccggg tcctctcttt      60

agggggaggt ccctgggccg aagcccgccc acctttcggt ggagccggac cgctttcgca     120

cccgtgctct ttcgagcaca cccaccccgt ttacgggggt ccctcggtca gctaccgagg     180

agagctcgct ggcccacact cctgagggaa tgtgggaacc ggcgttagcc actccgaagt     240

gcgtataacg cgcacgccgg cggacttcat gctgtcggtg atttcacctc cagtatggaa     300

ccacgctatg tagcgaccac tgtcgtgctt ttcgctgaag aacttgcgtt ctcgagcgat     360

acgagcaaga cggaaacccg aggtacgggt atccgcgagc agccgcccgt acggagtctt     420

ggtgtatacc gagactgccg taggcgggct gactacgtag tagtcggcag cgaggtccgt     480

cccaccgaag aacatcgaag gcacctggga ggagagccgt acccacacct tatagaggcg     540

tggatctgac atacctccga caactcccca accccgtagc cgatttaata tcagcatcag     600

ggcgaagaga ttgtcaacag gtttcttgat gtaaaacggt ttgacatcga caccacggta     660

aaagtgcgcg ccgcagctct cgcgaaagag cccggacacg aacgttttac gaagattcgg     720

tttaaaaccg tagtaggcaa gtgcctctag cacacggggt gcaatctcac tgggacatat     780

aatatcgtcc ccgtagatgc ctatggttcc ggcgttacca aaatggattt gggtcgcttt     840

gactattgcc cagaatatca tggactctag ctcaaatgtg aacccatttc ccattgtgga     900

aaatagttcc catcgtatcg tctcgccatc tacgattccg tagtgtgagc ggatacgatc     960

gagatatgaa tatagctctg gtgggagaaa actccacacc aggcgatcgg agatggaatc    1020

ggatgcagac gataagtcta tcgtcgcaag cgaaccatct acgctgccct gctgagccag    1080

acgctggttg atcgattgat cattcaggtc tataccaacg gatttgagcc ggcgtctgat    1140

gaaagcaccg acccctttct ggaggtacat attcatatca ggctccttac aggcagcccg    1200

atctatttta ttattcttcg gaactgtaaa cactccgttc cctacaacga gcctaaattc    1260

atatgactcg ttatagcgga ccgcgtgtct gatccacggc gcacattggt ctcggaccaa    1320

tagagccgct ctcagagcgc ggggggtaac ggttgcttgt tcagcgaact tcttgtaagg    1380

cgctgcatcc tgcaacttgt gccccatagg agcaccgttg gagaacgtgc attgcccaaa    1440

caacgacgat cggtagccag agaggaggtt gccaataagg ctacggatgc tggtttgtaa    1500

aacatccgga tcccatgaca aggatttgtc atgtaagaaa ccttctctat ttatctgacc    1560

gcgatcacca ttcgcctccc gtagcttagc gatagctaag gtacgacggg tcgcctcgtc    1620

attaccagaa cctaaggtcg gatgctttgt gagcaattcg tcccttaagt aagcaattgc    1680

tgtaaagtcg tcactgtgcg gatcaccgct tccagtagcg acagaagcaa ttgattggta    1740

aatttcgaga gaaagatcgc gaggaagatc aatacataaa gagttgaact tctttgttgt 1800

cttcgacatg ggtaatcctc atgtttgaat ggccggcgtc tattagtaga tgccggagtt 1860

tgctgcgatt gctgagggaa tcgggtttcc atcttttagg agaccttgca ttgccttaac 1920

aataagctcg cagtcggaat tcgtagcgaa aattggaatg gttagttcca tatttaagta 1980

cgaacgccat gcggctacag gaagctctac accaccaaca gtctgggttg ccactttagg 2040

cacctcgact ttgatggtgt atttgcgatt ctgcgcagag ctctgacgaa cgctacaggt 2100

tactttgtaa gcctgtgaac gcgagttaga gctgatccat tcagcgaccc cgttagcgaa 2160

gttgcttggg gcgacagtca cgtcgccagt tccgccattg tcgacgagaa cgaactgagt 2220

aaagttagaa gccatgcttc aaactccggt tgagggctct atctagagag ccgttgcctg 2280

attaatgcta acgcatctaa ggtatggacc atcgagaaag gagactttac gtacgcgcca 2340

gttgttggcc atacggattg tacccctcga tgcatggctg agatttgggc cttagcagtg 2400

ccctgtctct ccacagtcca cccgtaggga gcgtcaacgc ttatgatgga ctcacccgtt 2460

attacgtcag taactgttcc tgacatgtag gagcatccca cgggggccgt aaggccctcg 2520

agcatgttac ctacaggtag gagccagtcg acaacgaatg agaaaggcac cttttcccac 2580

actataccta gtgggttcaa gatacctaga gacgacaacc atgccaaacg tgcatcgttt 2640

atgtaaaacc atatcacgat acgtcgcgat atgttgcacg ttgtctggaa gtttgcagct 2700

ggatacgaca gacggccatc taacttgatg ttagtaccga cctgacgtac ggctctcata 2760

ggaagaaact cttgaaggtg aaccttcgta agcatctcat atgcaccctg gatatcactc 2820

attagtggta accaaccgaa ctgcaactcc aaccacctgc cggccacgtg ttttgatcga 2880

aactttcgat cttcgtttag ggcaaggtag cggagcgcct ggcgccaatt accgcgacga 2940

gcggcagtgt acgccttcac gagcgcaatg gtttgcgtcg cgagttgtga ggctgtcgac 3000

ctggcctctg ctaaagcaac accaaggtta aaattaccct gggtgacctt ttgcaggact 3060

tcggtcgacg cccggttcgc aacgttctgc ggcacttcga tgtaagtcaa gttttggctt 3120

acagggaaga ggctgtagca ggagcgtgcg tcgagggaga agccgaaacc ggctttctcc 3180

tcgtacgggc gaccccacga tgacccactt cgcttgtagg caccttgatc tatcgatgtg 3240

acacttaacg ccccccgtga atacggagag gggtagtgcc actgtttcgt tttggcccca 3300

gtcgagttaa aacgaccggg agtccagttc gaacgatatt ttaaagagaa tgagttatct 3360

tcagtctcac cgtccgcgta aacgcgaacg gaggggacga aggtctcgtt ctccctatca 3420

agggtactaa aagctcgcac aggtcaaacc tcctaggaat ggaattccgg ctacctacag 3480

cgatagccat ggtagcgtct cgctaaagac attaaaaatg gcattagctc gacaggaagt 3540

tgagcaggac cccgaaaggg gtcccaccc 3569

<210>  21
<211>  23
<212>  DNA
<213>  Artificial Sequence

<220>
<223>  MAW26 primer

<400>  21
ttggaaatgt ggaaaggaag gac                                              23


<210>  22
<211>  31
<212>  DNA
<213>  Artificial Sequence

<220>
<223>  RT21 primer

<400>  22
ctgtatttct gctattaagt cttttgatgg g                                     31


<210>  23
<211>  801
<212>  DNA
<213>  Saccharomyces cerevisiae

<400>  23
atgtcgaaag ctacatataa ggaacgtgct gctactcatc ctagtcctgt tgctgccaag      60

ctatttaata tcatgcacga aaagcaaaca aacttgtgtg cttcattgga tgttcgtacc     120

accaaggaat tactggagtt agttgaagca ttaggtccca aaatttgttt actaaaaaca     180

catgtggata tcttgactga tttttccatg gagggcacag ttaagccgct aaaggcatta     240

tccgccaagt acaatttttt actcttcgaa gacagaaaat ttgctgacat tggtaataca     300

gtcaaattgc agtactctgc gggtgtatac agaatagcag aatgggcaga cattacgaat     360

gcacacggtg tggtgggccc aggtattgtt agcggtttga agcaggcggc agaagaagta     420

acaaaggaac ctagaggcct tttgatgtta gcagaattgt catgcaaggg ctccctatct     480

actggagaat atactaaggg tactgttgac attgcgaaga gcgacaaaga ttttgttatc     540

ggctttattg ctcaaagaga catgggtgga agagatgaag gttacgattg gttgattatg     600

acacccggtg tgggtttaga tgacaaggga gacgcattgg gtcaacagta tagaaccgtg     660

gatgatgtgg tctctacagg atctgacatt attattgttg gaagaggact atttgcaaag     720

ggaagggatg ctaaggtaga gggtgaacgt tacagaaaag caggctggga agcatatttg     780

agaagatgcg gccagcaaaa c                                               801


<210>  24
<211>  804
<212>  DNA

<213>  Kluyveromyces lactis

<400>  24
atgtccacaa aatcatatac cagtagagct gagactcatg caagtccggt tgcatcgaaa      60

cttttacgtt taatggatga aaagaagacc aatttgtgtg cttctcttga cgttcgttcg     120

actgatgagc tattgaaact tgttgaaacg ttgggtccat acatttgcct tttgaaaaca     180

cacgttgata tcttggatga tttcagttat gagggtactg tcgttccatt gaaagcattg     240

gcagagaaat acaagttctt gatatttgag gacagaaaat tcgccgatat cggtaacaca     300

gtcaaattac aatatacatc gggcgtttac cgtatcgcag aatggtctga tatcaccaac     360

gcccacgggg ttactggtgc tggtattgtt gctggcttga aacaaggtgc gcaagaggtc     420

accaaagaac caaggggatt attgatgctt gctgaattgt cttccaaggg ttctctagca     480

cacggtgaat atactaaggg taccgttgat attgcaaaga gtgataaaga tttcgttatt     540

gggttcattg ctcagaacga tatgggagga agagaagaag ggtttgattg gctaatcatg     600

accccaggtg taggtttaga cgacaaaggc gatgcattgg gtcagcagta cagaaccgtc     660

gacgaagttg taagtggtgg atcagatatc atcattgttg gcagaggact tttcgccaag     720

ggtagagatc ctaaggttga aggtgaaaga tacagaaatg ctggatggga agcgtaccaa     780

aagagaatca gcgctcccca ttaa                                            804


<210>  25
<211>  7022
<212>  DNA
<213>  Artificial Sequence

<220>
<223>  pAmkPR

<400>  25
gtggtcttct acacagacaa gatgaaacaa ttcggcatta atacctgaga gcaggaagag      60

caagataaaa ggtagtattt gttggcgatc cccctagagt cttttacatc ttcggaaaac     120

aaaaactatt ttttctttaa tttctttttt tactttctat ttttaattta tatatttata     180

ttaaaaaatt taaattataa ttatttttat agcacgtgat gaaaaggacc ctaagaaacc     240

attattatca tgacattaac ctataaaaat aggcgtatca cgaggccctt tcgtctcgcg     300

cgtttcggtg atgacggtga aaacctctga cacatgcagc tcccggagac ggtcacagct     360

tgtctgtaag cggatgccgg gagcagacaa gcccgtcagg gcgcgtcagc gggtgttggc     420

gggtgtcggg gctggcttaa ctatgcggca tcagagcaga ttgtactgag agtgcaccat     480

aaattcccgt tttaagagct tggtgagcgc taggagtcac tgccaggtat cgtttgaaca     540

cggcattagt cagggaagtc ataacacagt cctttcccgc aattttcttt ttctattact     600

cttggcctcc tctagtacac tctatatttt tttatgcctc ggtaatgatt ttcatttttt     660

tttttccacc tagcggatga ctcttttttt ttcttagcga ttggcattat cacataatga 720

attatacatt atataaagta atgtgatttc ttcgaagaat atactaaaaa atgagcaggc 780

aagataaacg aaggcaaaga tgacagagca gaaagcccta gtaaagcgta ttacaaatga 840

aaccaagatt cagattgcga tctctttaaa gggtggtccc ctagcgatag agcactcgat 900

cttcccagaa aaagaggcag aagcagtagc agaacaggcc acacaatcgc aagtgattaa 960

cgtccacaca ggtatagggt ttctggacca tatgatacat gctctggcca agcattccgg 1020

ctggtcgcta atcgttgagt gcattggtga cttacacata gacgaccatc acaccactga 1080

agactgcggg attgctctcg gtcaagcttt taaagaggcc ctaggggccg tgcgtggagt 1140

aaaaaggttt ggatcaggat ttgcgccttt ggatgaggca ctttccagag cggtggtaga 1200

tctttcgaac aggccgtacg cagttgtcga acttggtttg caaagggaga aagtaggaga 1260

tctctcttgc gagatgatcc cgcattttct tgaaagcttt gcagaggcta gcagaattac 1320

cctccacgtt gattgtctgc gaggcaagaa tgatcatcac cgtagtgaga gtgcgttcaa 1380

ggctcttgcg gttgccataa gagaagccac ctcgcccaat ggtaccaacg atgttccctc 1440

caccaaaggt gttcttatgt agtgacaccg attatttaaa gctgcagcat acgatatata 1500

tacatgtgta tatatgtata cctatgaatg tcagtaagta tgtatacgaa cagtatgata 1560

ctgaagatga caaggtaatg catcattcta tacgtgtcat tctgaacgag gcgcgctttc 1620

ctttttcctt tttgcttttt cttttttttt ctcttgaact cgacggatct atgcggtgtg 1680

aaataccgca cagatgcgta aggagaaaat accgcatcag gaaattgtaa gcgttaatat 1740

tttgttaaaa ttcgcgttaa atttttgtta aatcagctca ttttttaacc aataggccga 1800

aatcggcaaa atcccttata aatcaaaaga atagaccgag atagggttga gtgttgttcc 1860

agtttggaac aagagtccac tattaaagaa cgtggactcc aacgtcaaag ggcgaaaaac 1920

cgtctatcag ggcgatggcc cactacgtga accatcaccc taatcaagtt ttttggggtc 1980

gaggtgccgt aaagcactaa atcggaaccc taaagggagc ccccgattta gagcttgacg 2040

gggaaagccg gcgaacgtgg cgagaaagga agggaagaaa gcgaaaggag cgggcgctag 2100

ggcgctggca agtgtagcgg tcacgctgcg cgtaaccacc acacccgccg cgcttaatgc 2160

gccgctacag ggcgcgtcca ttcgccattc aggctgcgca actgttggga agggcgatcg 2220

gtgcgggcct cttcgctatt acgccagctg gcgaaagggg ggatgtgctg caaggcgatt 2280

aagttgggta acgccagggt tttcccagtc acgacgttgt aaaacgacgg ccagtgagcg 2340

cgcgtaatac gactcactat agggggttga agtgagtgtt gcaccgtgcc aatgcaggtg 2400

gctattagat taaatatgtg atttgttcta ttaagtttcc tgtataatta atggggagcg 2460

ctgattctct tttggtacgc ttcccatcca gcatttctgt atctttcacc ttcaacctta 2520

ggatctctac ccttggcgaa aagtcctctg ccaacaatga tgatatctga tccaccactt 2580

acaacttcgt cgacggttct gtactgctga cccaatgcat cgcctttgtc gtctaaacct 2640

acacctgggg tcatgattag ccaatcaaac ccttcttctc ttcctcccat atcgttctga 2700

gcaatgaacc caataacgaa atctttatca ctctttgcaa tatcaacggt acccttagta 2760

tattcaccgt gtgctagaga acccttggaa gacaattcag caagcatcaa taatcccctt 2820

ggttctttgg tgacctcttg cgcaccttgt ttcaagccag caacaatacc agcaccagta 2880

accccgtggg cgttggtgat atcagaccat tctgcgatac ggtaaacgcc cgatgtatat 2940

tgtaatttga ctgtgttacc gatatcggcg aattttctgt cctcaaatat caagaacttg 3000

tatttctctg ccaatgcttt caatggaacg acagtaccct cataactgaa atcatccaag 3060

atatcaacgt gtgttttcaa aaggcaaatg tatggaccca acgtttcaac aagtttcaat 3120

agctcatcag tcgaacgaac gtcaagagaa gcacacaaat tggtcttctt ttcatccatt 3180

aaacgtaaaa gtttcgatgc aaccggactt gcatgagtct cagctctact ggtatatgat 3240

tttgtggaca tggtgcaact aattgacggg agtgtattga cgctggcgta ctggctttca 3300

caaaatggcc caatcacaac cacatcttag atagttgaaa tgactttaga taacatcaat 3360

tgagatgagc ttaatcagct cttaatccat tcctggcttt aattttactg gtacagtctc 3420

aataggactc gagggagttg ttgtctctac cccacatgga tccggggttt tttctccttg 3480

acgttaaagt atagaggtat attaacaatt ttttgttgat acttttatta catttgaata 3540

agaagtaata caaaccgaaa atgttgaaag tattagttaa agtggttatg cagtttttgc 3600

atttatatat ctgttaatag atcaaaaatc atcgcttcgc tgattaatta ccccagaaat 3660

aaggctaaaa aactaatcgc attatcatcc tatggttgtt aatttgattc gttcatttga 3720

aggtttgtgg ggccaggtta ctgccaattt ttcctcttca taaccataaa agctagtatt 3780

gtagaatctt tattgttcgg agcagtgcgg cgcgaggcac atctgcgttt caggaacgcg 3840

accggtgaag acgaggacgc acggaggaga gtcttccttc ggagggctgt cacccgctcg 3900

gcggcttcta atccgtactt caatatagca atgagcagtt aagcgtatta ctgaaagttc 3960

caaagagaag gtttttttag gctaagataa tggggctctt tacatttcca caacatataa 4020

gtaagattag atatggatat gtatatggat atgtatatgg tggtaatgcc atgtaatatg 4080

attattaaac ttctttgcgt ccatccaaaa aaaaagtaag aatttttgaa aattcaatat 4140

aaatgacagc tcagttacaa agtgaaagta cttctaaaat tgttttggtt acaggtggtg 4200

ctggatacat tggttcacac actgtggtag agctaattga gaatggatat gactgtgttg 4260

ttgctgataa cctgtcgaat tccatggtca tagctgtttc ctgtgtgaaa ttgttatccg 4320

ctcacaattc cacacaacat acgagccgga agcataaagt gtaaagcctg gggtgcctaa 4380

tgagtgagct aactcacatt aattgcgttg cgctcactgc ccgctttcca gtcgggaaac 4440

ctgtcgtgcc agctgcatta atgaatcggc caacgcgcgg ggagaggcgg tttgcgtatt 4500

gggcgctctt ccgcttcctc gctcactgac tcgctgcgct cggtcgttcg gctgcggcga 4560

gcggtatcag ctcactcaaa ggcggtaata cggttatcca cagaatcagg ggataacgca 4620

ggaaagaaca tgtgagcaaa aggccagcaa aaggccagga accgtaaaaa ggccgcgttg 4680

ctggcgtttt tccataggct ccgcccccct gacgagcatc acaaaaatcg acgctcaagt 4740

cagaggtggc gaaacccgac aggactataa agataccagg cgtttccccc tggaagctcc 4800

ctcgtgcgct ctcctgttcc gaccctgccg cttaccggat acctgtccgc ctttctccct 4860

tcgggaagcg tggcgctttc tcatagctca cgctgtaggt atctcagttc ggtgtaggtc 4920

gttcgctcca agctgggctg tgtgcacgaa ccccccgttc agcccgaccg ctgcgcctta 4980

tccggtaact atcgtcttga gtccaacccg gtaagacacg acttatcgcc actggcagca 5040

gccactggta acaggattag cagagcgagg tatgtaggcg gtgctacaga gttcttgaag 5100

tggtggccta actacggcta cactagaagg acagtatttg gtatctgcgc tctgctgaag 5160

ccagttacct tcggaaaaag agttggtagc tcttgatccg gcaaacaaac caccgctggt 5220

agcggtggtt tttttgtttg caagcagcag attacgcgca gaaaaaaagg atctcaagaa 5280

gatcctttga tcttttctac ggggtctgac gctcagtgga acgaaaactc acgttaaggg 5340

attttggtca tgagattatc aaaaaggatc ttcacctaga tccttttaaa ttaaaaatga 5400

agttttaaat caatctaaag tatatatgag taaacttggt ctgacagtta ccaatgctta 5460

atcagtgagg cacctatctc agcgatctgt ctatttcgtt catccatagt tgcctgactc 5520

cccgtcgtgt agataactac gatacgggag ggcttaccat ctggccccag tgctgcaatg 5580

ataccgcgag acccacgctc accggctcca gatttatcag caataaacca gccagccgga 5640

agggccgagc gcagaagtgg tcctgcaact ttatccgcct ccatccagtc tattaattgt 5700

tgccgggaag ctagagtaag tagttcgcca gttaatagtt tgcgcaacgt tgttgccatt 5760

gctacaggca tcgtggtgtc acgctcgtcg tttggtatgg cttcattcag ctccggttcc 5820

caacgatcaa ggcgagttac atgatccccc atgttgtgca aaaaagcggt tagctccttc 5880

ggtcctccga tcgttgtcag aagtaagttg gccgcagtgt tatcactcat ggttatggca 5940

gcactgcata attctcttac tgtcatgcca tccgtaagat gcttttctgt gactggtgag 6000

tactcaacca agtcattctg agaatagtgt atgcggcgac cgagttgctc ttgcccggcg 6060

tcaatacggg ataataccgc gccacatagc agaactttaa aagtgctcat cattggaaaa 6120

cgttcttcgg ggcgaaaact ctcaaggatc ttaccgctgt tgagatccag ttcgatgtaa 6180

cccactcgtg cacccaactg atcttcagca tcttttactt tcaccagcgt ttctgggtga 6240

gcaaaaacag gaaggcaaaa tgccgcaaaa aagggaataa gggcgacacg gaaatgttga 6300

atactcatac tcttcctttt tcaatattat tgaagcattt atcagggtta ttgtctcatg 6360

agcggataca tatttgaatg tatttagaaa aataaacaaa taggggttcc gcgcacattt 6420

ccccgaaaag tgccacctgg gtccttttca tcacgtgcta taaaaataat tataatttaa 6480

attttttaat ataaatatat aaattaaaaa tagaaagtaa aaaaagaaat taaagaaaaa 6540

atagtttttg ttttccgaag atgtaaaaga ctctaggggg atcgccaaca aatactacct 6600

tttatcttgc tcttcctgct ctcaggtatt aatgccgaat tgtttcatct tgtctgtgta 6660

gaagaccaca cacgaaaatc ctgtgatttt acattttact tatcgttaat cgaatgtata 6720

tctatttaat ctgcttttct tgtctaataa atatatatgt aaagtacgct ttttgttgaa 6780

attttttaaa cctttgttta tttttttttc ttcattccgt aactcttcta ccttctttat 6840

ttactttcta aaatccaaat acaaaacata aaaataaata aacacagagt aaattcccaa 6900

attattccat cattaaaaga tacgaggcgc gtgtaagtta caggcaagcg atccgtccta 6960

agaaaccatt attatcatga cattaaccta taaaaatagg cgtatcacga ggccctttcg 7020

tc 7022

<210> 26
<211> 7980
<212> DNA
<213> Artificial Sequence

<220>
<223> pAmkRT

<400> 26
gacgaaaggg cctcgtgata cgcctatttt tataggttaa tgtcatgata ataatggttt 60

cttagtatga tccaatatca aaggaaatga tagcattgaa ggatgagact aatccaattg 120

aggagtggca gcatatagaa cagctaaagg gtagtgctga aggaagcata cgataccccg 180

catggaatgg gataatatca caggaggtac tagactacct ttcatcctac ataaatagac 240

gcatataagt acgcatttaa gcataaacac gcactatgcc gttcttctca tgtatatata 300

tatacaggca acacgcagat ataggtgcga cgtgaacagt gagctgtatg tgcgcagctc 360

gcgttgcatt ttcggaagcg ctcgttttcg gaaacgcttt gaagttccta ttccgaagtt 420

cctattctct agaaagtata ggaacttcag agcgcttttg aaaaccaaaa gcgctctgaa 480

gacgcacttt caaaaaacca aaaacgcacc ggactgtaac gagctactaa aatattgcga 540

ataccgcttc cacaaacatt gctcaaaagt atctctttgc tatatatctc tgtgctatat 600

ccctatataa cctacccatc cacctttcgc tccttgaact tgcatctaaa ctcgacctct 660

acatttttta tgtttatctc tagtattact ctttagacaa aaaaattgta gtaagaacta 720

ttcatagagt gaatcgaaaa caatacgaaa atgtaaacat ttcctatacg tagtatatag 780

agacaaaata gaagaaaccg ttcataattt tctgaccaat gaagaatcat caacgctatc 840

actttctgtt cacaaagtat gcgcaatcca catcggtata gaatataatc ggggatgcct 900

ttatcttgaa aaaatgcacc cgcagcttcg ctagtaatca gtaaacgcgg gaagtggagt 960

caggcttttt ttatggaaga gaaaatagac accaaagtag ccttcttcta accttaacgg 1020

acctacagtg caaaaagtta tcaagagact gcattataga gcgcacaaag gagaaaaaaa 1080

gtaatctaag atgctttgtt agaaaaatag cgctctcggg atgcattttt gtagaacaaa 1140

aaagaagtat agattctttg ttggtaaaat agcgctctcg cgttgcattt ctgttctgta 1200

aaaatgcagc tcagattctt tgtttgaaaa attagcgctc tcgcgttgca tttttgtttt 1260

acaaaaatga agcacagatt cttcgttggt aaaatagcgc tttcgcgttg catttctgtt 1320

ctgtaaaaat gcagctcaga ttctttgttt gaaaaattag cgctctcgcg ttgcattttt 1380

gttctacaaa atgaagcaca gatgcttcgt tcaggtggca cttttcgggg aaatgtgcgc 1440

ggaaccccta tttgtttatt tttctaaata cattcaaata tgtatccgct catgagacaa 1500

taaccctgat aaatgcttca ataatattga aaaaggaaga gtatgagtat tcaacatttc 1560

cgtgtcgccc ttattccctt ttttgcggca ttttgccttc ctgtttttgc tcacccagaa 1620

acgctggtga aagtaaaaga tgctgaagat cagttgggtg cacgagtggg ttacatcgaa 1680

ctggatctca acagcggtaa gatccttgag agttttcgcc ccgaagaacg ttttccaatg 1740

atgagcactt ttaaagttct gctatgtggc gcggtattat cccgtattga cgccgggcaa 1800

gagcaactcg gtcgccgcat acactattct cagaatgact tggttgagta ctcaccagtc 1860

acagaaaagc atcttacgga tggcatgaca gtaagagaat tatgcagtgc tgccataacc 1920

atgagtgata acactgcggc caacttactt ctgacaacga tcggaggacc gaaggagcta 1980

accgcttttt tgcacaacat gggggatcat gtaactcgcc ttgatcgttg ggaaccggag 2040

ctgaatgaag ccataccaaa cgacgagcgt gacaccacga tgcctgtagc aatggcaaca 2100

acgttgcgca aactattaac tggcgaacta cttactctag cttcccggca acaattaata 2160

gactggatgg aggcggataa agttgcagga ccacttctgc gctcggccct tccggctggc 2220

tggtttattg ctgataaatc tggagccggt gagcgtgggt ctcgcggtat cattgcagca 2280

ctggggccag atggtaagcc ctcccgtatc gtagttatct acacgacggg gagtcaggca 2340

actatggatg aacgaaatag acagatcgct gagataggtg cctcactgat taagcattgg 2400

taactgtcag accaagttta ctcatatata ctttagattg atttaaaact tcatttttaa 2460

tttaaaagga tctaggtgaa gatccttttt gataatctca tgaccaaaat cccttaacgt 2520

gagttttcgt tccactgagc gtcagacccc gtagaaaaga tcaaaggatc ttcttgagat 2580

cctttttttc tgcgcgtaat ctgctgcttg caaacaaaaa aaccaccgct accagcggtg 2640

gtttgtttgc cggatcaaga gctaccaact ctttttccga aggtaactgg cttcagcaga 2700

gcgcagatac caaatactgt ccttctagtg tagccgtagt taggccacca cttcaagaac 2760

tctgtagcac cgcctacata cctcgctctg ctaatcctgt taccagtggc tgctgccagt 2820

ggcgataagt cgtgtcttac cgggttggac tcaagacgat agttaccgga taaggcgcag 2880

cggtcgggct gaacgggggg ttcgtgcaca cagcccagct tggagcgaac gacctacacc 2940

gaactgagat acctacagcg tgagctatga gaaagcgcca cgcttcccga agggagaaag 3000

gcggacaggt atccggtaag cggcagggtc ggaacaggag agcgcacgag ggagcttcca 3060

gggggaaacg cctggtatct ttatagtcct gtcgggtttc gccacctctg acttgagcgt 3120

cgatttttgt gatgctcgtc aggggggcgg agcctatgga aaaacgccag caacgcggcc 3180

tttttacggt tcctggcctt ttgctggcct tttgctcaca tgttctttcc tgcgttatcc 3240

cctgattctg tggataaccg tattaccgcc tttgagtgag ctgataccgc tcgccgcagc 3300

cgaacgaccg agcgcagcga gtcagtgagc gaggaagcgg aagagcgccc aatacgcaaa 3360

ccgcctctcc ccgcgcgttg gccgattcat taatgcagct ggcacgacag gtttcccgac 3420

tggaaagcgg gcagtgagcg caacgcaatt aatgtgagtt acctcactca ttaggcaccc 3480

caggctttac actttatgct tccggctcct atgttgtgtg gaattgtgag cggataacaa 3540

tttcacacag gaaacagcta tgaccatgat tacgccaagc gcgcaattaa ccctcactaa 3600

agggaacaaa agctggagct cagtttatca ttatcaatac tcgccatttc aaagaatacg 3660

taaataatta atagtagtga ttttcctaac tttatttagt caaaaaatta gccttttaat 3720

tctgctgtaa cccgtacatg cccaaaatag ggggcgggtt acacagaata tataacatcg 3780

taggtgtctg ggtgaacagt ttattcctgg catccactaa atataatgga gcccgctttt 3840

taagctggca tccagaaaaa aaaagaatcc cagcaccaaa atattgtttt cttcaccaac 3900

catcagttca taggtccatt ctcttagcgc aactacagag aacaggggca caaacaggca 3960

aaaaacgggc acaacctcaa tggagtgatg caacctgcct ggagtaaatg atgacacaag 4020

gcaattgacc cacgcatgta tctatctcat tttcttacac cttctattac cttctgctct 4080

ctctgatttg gaaaaagctg aaaaaaaagg ttgaaaccag ttccctgaaa ttattcccct 4140

acttgactaa taagtatata aagacggtag gtattgattg taattctgta aatctatttc 4200

ttaaacttct taaattctac ttttatagtt agtctttttt ttagttttaa aacaccagaa 4260

cttagtttcg acggattcta gaactagtat gagatttcct tcaattttta ctgcagtttt 4320

attcgcagca tcctccgcat tagctgctcc agtcaacact acaacagaag atgaaacggc 4380

acaaattccg gctgaagctg tcatcggtta cttagattta gaaggggatt tcgatgttgc 4440

tgttttgcca ttttccaaca gcacaaataa cgggttattg tttataaata ctactattgc 4500

cagcattgct gctaaagaag aaggggtatc tttggataaa agagaggctg aagcttggca 4560

ttggttgcaa ctaaaacctg gccaaccaat gtacaagaga gaagccgaag ctgaagctcc 4620

cattagccct attgagactg taccagtaaa attaaagccc gggatggatg gtcagattta 4680

cccatggatt aaagtaaggc aattatgtaa actccttaga ggaaccaaag cactaacaga 4740

agtaatacca ctaacagaag aagcagagct agaactggca gaaaacagag agattctaaa 4800

agaaccagta catggagtgt attatgaccc atcaaaagac ttaatagcag aaatccagaa 4860

gcaggggcaa ggcccaatgg acatatcaaa tttatcaaga gccatttaaa aatctgaaaa 4920

caggaaaata tgcaagaacg aggggtgccc acactaatga tgtaaaacaa ttaacagagg 4980

cagtgcaaaa aataaccaca gaaagcatag taatatgggg aaagactcct aaatttaaac 5040

tacccataca aaaggaaaca tgggaaacat ggtggacaga gtattggcaa gccacctgga 5100

ttcctgagtg ggagtttgtc aatacccctc ctttagtgaa attatggtac cagttagaga 5160

aagaacccat agtaggagca gaaacgttct atgtagatgg ggcagctagc agggagacta 5220

aattaggaaa agcaggatat gttactaata gaggaagaca aaaagttgtc accctaactg 5280

acacaacaaa tcagaagact gagttacaag caattcatct agctttgcag gattcgggat 5340

tagaagtaaa tatagtaaca gactcacaat atgcattagg aatcattcaa gcacaaccag 5400

ataaaagtga atcagagtta gtcaatcaaa taatagagca gttaataaaa aaggaaaagg 5460

tctatctggc atgggtacca gcacacaaag gaattggagg aaatgaacaa gtagataaat 5520

tagtcagtgc tggaatcagg aaagtactat agtaagctta tcgataccgt cgacctcgag 5580

tcatgtaatt agttatgtca cgcttacatt cacgccctcc ccccacatcc gctctaaccg 5640

aaaaggaagg agttagacaa cctgaagtct aggtccctat ttattttttt atagttatgt 5700

tagtattaag aacgttattt atatttcaaa tttttctttt ttttctgtac agacgcgtgt 5760

acgcatgtaa cattatactg aaaaccttgc ttgagaaggt tttgggacgc tcgaaggctt 5820

taatttgcgg ccggtaccca attcgcccta tagtgagtcg tattacgcgc gctcactggc 5880

cgtcgtttta caacgtcgtg actgggaaaa ccctggcgtt acccaactta atcgccttgc 5940

agcacatccc cctttcgcca gctggcgtaa tagcgaagag gcccgcaccg atcgcccttc 6000

ccaacagttg cgcagcctga atggcgaatg gcgcgacgcg ccctgtagcg gcgcattaag 6060

cgcggcgggt gtggtggtta cgcgcagcgt gaccgctaca cttgccagcg ccctagcgcc 6120

cgctcctttc gctttcttcc cttcctttct cgccacgttc gccggctttc cccgtcaagc 6180

tctaaatcgg gggctccctt tagggttccg atttagtgct ttacggcacc tcgaccccaa 6240

aaaacttgat tagggtgatg gttcacgtag tgggccatcg ccctgataga cggtttttcg 6300

ccctttgacg ttggagtcca cgttctttaa tagtggactc ttgttccaaa ctggaacaac 6360

actcaaccct atctcggtct attcttttga tttataaggg attttgccga tttcggccta 6420

ttggttaaaa aatgagctga tttaacaaaa atttaacgcg aattttaaca aaatattaac 6480

gtttacaatt tcctgatgcg gtattttctc cttacgcatc tgtgcggtat ttcacaccgc 6540

atagatcggc aagtgcacaa acaatactta aataaatact actcagtaat aacctatttc 6600

ttagcatttt tgacgaaatt tgctattttg ttagagtctt ttacaccatt tgtctccaca 6660

cctccgctta catcaacacc aataacgcca tttaatctaa gcgcatcacc aacattttct 6720

ggcgtcagtc caccagctaa cataaaatgt aagctttcgg ggctctcttg ccttccaacc 6780

cagtcagaaa tcgagttcca atccaaaagt tcacctgtcc cacctgcttc tgaatcaaac 6840

aagggaataa acgaatgagg tttctgtgaa gctgcactga gtagtatgtt gcagtctttt 6900

ggaaatacga gtcttttaat aactggcaaa ccgaggaact cttggtattc ttgccacgac 6960

tcatctccat gcagttggac gatatcaatg ccgtaatcat tgaccagagc caaaacatcc 7020

tccttaggtt gattacgaaa cacgccaacc aagtatttcg gagtgcctga actattttta 7080

tatgctttta caagacttga aattttcctt gcaataaccg ggtcaattgt tctctttcta 7140

ttgggcacac atataatacc cagcaagtca gcatcggaat ctagagcaca ttctgcggcc 7200

tctgtgctct gcaagccgca aactttcacc aatggaccag aactacctgt gaaattaata 7260

acagacatac tccaagctgc ctttgtgtgc ttaatcacgt atactcacgt gctcaatagt 7320

caccaatgcc ctccctcttg gccctctcct tttctttttt cgaccgaatt aattcttaat 7380

cggcaaaaaa agaaaagctc cggatcaaga ttgtacgtaa ggtgacaagc tatttttcaa 7440

taaagaatat cttccactac tgccatctgg cgtcataact gcaaagtaca catatattac 7500

gatgctgttc tattaaatgc ttcctatatt atatatatag taatgtcgtt gatctatggt 7560

gcactctcag tacaatctgc tctgatgccg catagttaag ccagccccga cacccgccaa 7620

cacccgctga cgcgccctga cgggcttgtc tgctcccggc atccgcttac agacaagctg 7680

tgaccgtctc cgggagctgc atgtgtcaga ggttttcacc gtcatcaccg aaacgcgcga 7740

gacgaaaggg cctcgtgata cgcctatttt tataggttaa tgtcatgaaa gtggtatggt 7800

gcactctcag tacaatctgc tctgatgccg catagttaag ccagccccga cacccgccaa 7860

cacccgctga cgcgccctga cgggcttgtc tgctcccggc atccgcttac agacaagctg 7920

tgaccgtctc cgggagctgc atgtgtcaga ggttttcacc gtcatcaccg aaacgcgcga 7980

<210> 27
<211> 44
<212> DNA
<213> Artificial Sequence

<220>
<223> HIV2 RT 5′ primer

<400> 27
caagagagaa gccgaagctg aagctccagt cgccaagtta gacc 44

<210> 28

<211>  23
<212>  DNA
<213>  Artificial Sequence

<220>
<223>  HIV2 RT 3' primer

<400>  28
aaaggtctct gtgcctggta tag                                              23


<210>  29
<211>  28
<212>  DNA
<213>  Artificial Sequence

<220>
<223>  A35 primer

<400>  29
ttggttgcac tttaaatttt cccattag                                         28


<210>  30
<211>  58
<212>  DNA
<213>  Artificial Sequence

<220>
<223>  HIV2 PR 5' primer

<400>  30
tatactttaa cgtcaaggag aaaaaacccc ggatccatgg cgacagagga cttgctac        58


<210>  31
<211>  44
<212>  DNA
<213>  Artificial Sequence

<220>
<223>  HIV2 PR 3' primer

<400>  31
tgagcttaat cagctcttaa tccatggtct aacttggcga ctgg                       44


<210>  32
<211>  298
<212>  DNA
<213>  Human immunodeficiency virus type 2

<400>  32
acctcaattc tctctttgga aaagaccagt agtcacagca ttcatcgagg atcagccggt      60

agaagtctta ctagacacag gagctgatga ctcaatagta gcaggaatag agttagggga     120

caattacact ccaaaaatag tggggggaat agggggattc ataaatacca aagaatataa     180

aaatgtagaa ataaaggtac taaataaaag agtaagagcc accataatga caggagatac     240

cccaatcaac atttttggca gaaatattct ggcaacctta ggcatgtcat taaaccta       298

## Description

### FIELD OF THE INVENTION

The present invention relates to methods for identifying antiretroviral compounds targeting the reverse transcriptase of HIV in order to better manage therapeutic strategies, and kits for the implementation of said methods.

### BACKGROUND OF THE INVENTION

According to the WHO data, a third of the 35 million people living with HIV at the end of 2013 receives Anti-Retroviral Therapy (ART). Despite the huge progress in drug formulation, HIV drug resistance has been reported for each new antiretroviral drug introduced. The emergence and spread of high level of HIV drug resistance could compromise the effectiveness of national HIV treatment programs.

Different approaches are used to test drug resistance. One of them consists in monitoring the viral load of HIV infected patient every 12 months from the beginning of ART. A sudden increase of HIV viral load is generally indicative of the emergence of a specific mutation in the genome of HIV. In some case, late diagnosis of treatment failure is associated with accumulated drug resistance mutations and high level cross resistance to subsequent regimens. The main drawback of this technic is that mutations are not identified.

ART is based on the use of compounds targeting specifically the reverse transcriptase (RT), the protease (PR), the integrase or other targets related to the entry of the virus within the cell. Considering RT, the compounds that inhibit HIV RT are subdivided in three main subclasses: the nucleoside RT inhibitors (NRTIs), the nucleotide RT inhibitors (NtRTIs) and the non-nucleoside RT inhibitors (NNRTIs).

The follow up of HIV infected patient can also be performed by genotypying. This technic allows the identification of already known functional mutations within the nucleic acid sequence coding for the viral proteins notably by bulk sequencing, oligonucleotide ligation assay, line probe assay, microarray hybridization, heteroduplex formation, allele specific PCR and single genome sequencing. All these technics enable the identification of signature mutations for patient receiving nucleoside analogue and non analogue reverse transcriptase inhibitor (NRTI, and NNRTI) as well as nucleotide RT inhibitors (NtRTIs) treatments. As an example, multi-NRTI resistance can be associated with the T69 insertion complex and the Q151M complex. The Q151 complex is a cluster of mutations including A62V, V75I, F77L, F116Y, and Q151M, which affects all current NRTIs except tenofovir. Q151M is much more frequently selected in HIV-2 infection. The T69 complex includes a substitution at codon 69 (typically T69S), plus an insertion of two or more amino acids at codon 69, and may also include mutations M41L, A62V, K70R, L210W, T215Y/F, and K219Q/E. The T69 complex affects all currently approved NRTIs. A third NRTI class resistance signature is the T215Y mutation, plus a single change at codon 67, plus a double insertion between codons 68 and 69. People with this pattern have shown phenotypic resistance to AZT, 3TC, ddI, and d4T despite the absence of other mutations usually associated with these agents. A fourth NRTI class-resistance complex is M184V, L214F and R211K. This complex has been seen in four of 22 people who experienced treatment failure while taking 3TC and d4T. Genotypying technic is a cost effective technic which can not be performed to screen for new mutations by a systematic sequencing of the complete HIV genome. Moreover, it does not always allow the detection of low frequency drug resistant variants.

In order to monitor HIV drug resistance, phenotypic assays were developed. The first ones used viral vectors that were propagated in host cells. By measuring the cumulative phenotype of a viral population, these assays were used as predictors of the response to a particular treatment regimen. Now, this type of assay does not monitor the phenotype of individual virus genomes, and the phenotype of minor variants in the viral population may be obscured. Consequently, population based phenotypic assays detect drug resistance only when resistant HIV variants comprise a significant portion of the viral population.

A phenotypic assay based on the hybrid elements derived from yeast Ty1 retrotransposon in which reverse transcriptase is provided by HIV RT has been developed by NISSLEY et al. (Journal of clinical microbiology, vol.43(11), p:5696-5704, 2005). By using this assay, the frequency of variants in a population and the detection of NNRTIs resistant variants were possible before they become a significant portion of the population. Nevertheless, TyHRT assay does not permit the identification of NRTI or NtNRTI resistant variants since these compounds can not cross the yeast membrane or can not be phosphorylated so as to be activated.

More recently, a phenotypic assay directly based on the assessment of the HIV RT was performed on patients infected by different HIV-1 subtypes and treated or not with NNRTIs or other drugs (AGNESKOG et al., PLOS One, vol. 9(7), e110508, 2014). The RT enzyme was directly extracted by lysing purified intact virions from plasma samples. Its activity was evaluated in presence of etravirine (ETR), a NNRTI compound, and compared to BH10-wild-type RT and its L100I mutant form. This assay allows assessment of resistance to ETR. Now the RT amount present in the plasma of the patients can be a limiting step of this technic. Moreover, this assay only represents the average RT activity.

Despite all the existing genotypic and phenotypic assays, there is a need for elaborating new methods for screening antiretroviral compounds more effective in treating patient, especially whose having mutated form of HIV resulting from the first line antiretroviral treatment. Moreover, the currently used tri- or tetra drug therapeutic strategy raised the question of identifying combination of compounds targeting more than one gene within the HIV machinery.

The inventors previously provided a method for determining sensitivity or resistance of HIV isolates to viral protease inhibitors (*See.* EP 2 408 914 B1). Now, the inventors propose a new method for assaying both HIV RT and HIV PR activities in the same assay. Surprisingly, the inventors have discovered that HIV RT can be efficiently tested directly in the extracellular media of transformed yeast expressing the HIV RT, without performing any purification step and/or using protease inhibitor to limit its degradation by yeast enzyme.

### SUMMARY OF THE INVENTION

The present invention is directed to a new method for identifying a compound inhibiting the activity of a HIV reverse transcriptase (HIV RT) and, optionally, a compound inhibiting the activity of a HIV protease (HIV PR) from the same HIV virus. This method permits to screen for new potential HIV RT and HIV PR inhibitors, and also to adapt the therapeutic protocol of a patient infected by HIV so as to obtain the best therapeutic response.

Accordingly, a first object of the invention is directed to a method for identifying a compound inhibiting HIV reverse transcriptase (HIV RT), said method comprising the steps of:
a) Transforming a yeast cell by at least one nucleic acid encoding for a HIV RT and a signal peptide, wherein the nucleic acid sequence encoding for HIV RT is under the control of a promoter and the signal peptide enables the secretion of the HIV RT in the supernatant;
b) Culturing the transformed yeast cell of step a) for secretion of the HIV RT in the supernatant;
c) Separating the cultured transformed yeast cells of step b) from the supernatant for collecting said supernatant containing the HIV RT;
d) Assaying the activity of said HIV RT in the collected supernatant of step c) in the presence or absence of a compound; and
e) Selecting a compound inhibiting HIV RT activity in step d).

In a preferred embodiment, the at least one nucleic acid encoding for HIV RT is related to type 1 or type 2 HIV and could be obtained from a subject sample, preferably a human patient infected by HIV-1 or HIV-2.

In a more preferred embodiment, the at least one nucleic acid encoding for HIV RT is amplified with a primer pair selected in the group comprising SEQ ID NO: 29 and SEQ ID NO: 5 or SEQ ID NO: 6 and SEQ ID NO: 7. Additional pair of primers can be simply designed by the skilled person from the consensus sequence of HIV-1 RT (from all HIV-1 sequences available in Los Alamos database) which is illustrated by SEQ ID NO: 8.

In still a more preferred embodiment, the method of the invention is for further identifying a compound inhibiting HIV protease (HIV PR), wherein:
- the step a) further comprises the transforming of said yeast cell by at least one nucleic acid encoding for a HIV PR, wherein the nucleic acid sequence encoding for HIV PR is under the control of a promoter;
- the step b) is for obtaining enough transformed yeast cells for testing HIV PR activity, and
- the step c) of separating the cultured transformed yeast cells from the supernatant is also for harvesting the cultured transformed yeast cell of step b); and
   wherein said method further comprises the steps:
   f) Assaying the activity of said HIV PR in the cultured transformed yeast cells in the presence or absence of a compound; and
   g) Selecting a compound inhibiting HIV PR activity in step f).

In this preferred embodiment, the at least one nucleic acid encoding for HIV PR is amplified with a primer pair selected in the group comprising SEQ ID NO: 9 and SEQ ID NO: 10 or SEQ ID NO: 9 and SEQ ID NO: 11. Additional pair of primers can be simply designed by the skilled person from the consensus sequence of HIV-1 PR (from all HIV-1 sequences available in Los Alamos database) which is illustrated by SEQ ID NO: 13.

Finally, an object of the invention is directed to a kit for identifying a compound inhibiting HIV RT, wherein said kit comprises:
(i) A yeast cell suitable for transformation,
(ii) A set of primers, and eventually a vector, so as to obtain a nucleic acid encoding for a HIV RT and a peptide signal.

In a preferred embodiment, the said kit further comprises:
(iii) A set of primers, and eventually a vector, so as to obtain a nucleic acid encoding for a HIV PR,
wherein the sets of primers in ii) and iii) being identical or distinct.

### BRIEF DESCRIPTION OF THE DRAWINGS

Figure 1 is a schematic representation of the co-transformation of the vector pAmkRT with the product of the nested PCR to obtain a transformed yeast cell having the sequence of the HIV RT under the control of the promoter GPD.
Figure 2 is a schematic representation of the secretion of HIV RT in the culture medium by the pAmkRT transformed yeast cell.
Figures 3 and 4 are schematic representation of the *in vitro* activity assay performed on secreted HIV RT. Newly synthetized cDNA including DIG-dUTP is detected by using anti-DIG-HRP monoclonal antibody allowing direct quantification of RT activity.
Figures 5 and 6 are maps of pAmkRT and pAmkPR vectors respectively.
Figure 7 shows a graph of RT activity inhibition as a function of inhibitor concentration. This graph illustrates the case where reverse transcriptase activity resulting from HIV infected patients (Patient #) is compared to a reference HIV reverse transcriptase already known to be sensitive to the RT inhibitor. Patient #1 is infected by HIV isolates more sensitive to the RT inhibitor compared to the reference. By contrast, Patient #2 is infected by HIV isolates more resistant to the RT inhibitor compared to the reference.
Figure 8 is illustrative of the efficiency of the signal peptide used for the correct level of secretion of HIV RT by transformed yeast cell. Two different signal peptide sequences, from pre-pro-MFα from alpha mating factor gene or pre-pro from alpha mating factor gene deleted of the MFα sequence were added at the amino terminal of the HIV RT and the HIV RT level of secretion was compared with the same construct lacking signal peptide.
Figure 9 shows a graph of RT activity inhibition as a function of inhibitor concentration. The Reverse Transcriptase reference comes from the isolate HIV-1 IIIB; this reference is sensitive to nevirapine (NVP), a NNRTI inhibitor. Three patients infected by HIV-1 were assayed by the method of the invention. Increasing concentrations of NVP allow the determination of IC50 values.

### DETAILED DESCRIPTION OF THE INVENTION

A first object of the invention relates to a method, preferably an *in vitro* method, for identifying a compound inhibiting HIV reverse transcriptase (HIV RT), said method comprising the steps of:
a) Transforming a yeast cell by at least one nucleic acid encoding for a HIV RT and a signal peptide, wherein the nucleic acid sequence encoding for HIV RT is under the control of a promoter and the signal peptide enables the secretion of the HIV RT in the supernatant;
b) Culturing the transformed yeast cell of step a) for secretion of the HIV RT in the supernatant;
c) Separating the cultured transformed yeast cells of step b) from the supernatant for collecting said supernatant containing the HIV RT;
d) Assaying the activity of said HIV RT in the collected supernatant of step c) in the presence or absence of a compound; and
e) Selecting a compound inhibiting HIV RT activity in step d).

The tested compound may be selected from a group comprising the molecules of a library, chemical molecules, natural molecules and molecules extracted from plants.

The term "HIV reverse transcriptase" refers to a protein, which is critical for the replication of the human immunodeficiency viruses and has three activities corresponding to a RNA-dependent DNA polymerase activity, a RNase H activity and a DNA-dependent DNA polymerase activity. Now, HIV is divided in two HIV types (HIV-1 and HIV-2), the HIV-1 type being further divided in four groups (M, N, O and P), and the HIV-1 group M being further classified into 9 distinct subtypes and inter-subtype circulating recombinant forms (CRFs). Depending on the HIV type, RT structure may vary. For HIV-1, RT is a heterodimer consisting of two polypeptide chains p66 (66kDa; 560 amino acid residues) and p51 (51 kDa; 440 amino acid residues), and for HIV-2, RT is a heterodimer consisting of two polypeptide chains p68 (68kDa; 559 amino acid residues) and p58 (58 kDa; 484 amino acid residues).

A consensus sequence of HIV-1 RT corresponding to all HIV-1 sequences available in Los Alamos database is illustrated by SEQ ID NO: 8.

Also, a consensus sequence of HIV-1 Pol corresponding to all HIV-1 sequences available in Los Alamos database is illustrated by SEQ ID NO: 12.

A sequence of HIV-2 RT available in NCBI database is illustrated by SEQ ID NO: 14. This sequence is from HIV-2 D194 isolate.

The nucleic acid encoding for a reverse transcriptase may be obtained from a biological sample. By "biological sample" is meant whole blood sample, serum, plasma, isolated PBMC or any biological fluid or organ infected by HIV.

In the method of the invention, the transformed host cell is a yeast cell and is selected from the group comprising *Saccharomyces cerevisiae, Candida albicans, Candida maltosa, Hansenula polymorpha, Kluyveromyces fragilis, Kluyveromyces lactis, Pichia guillerimondi, Pichia pastoris, Schizosaccharomyces pombe* and *Yarrowia lipolytica.* Preferably, the transformed yeast cell is *Saccharomyces cerevisiae.*

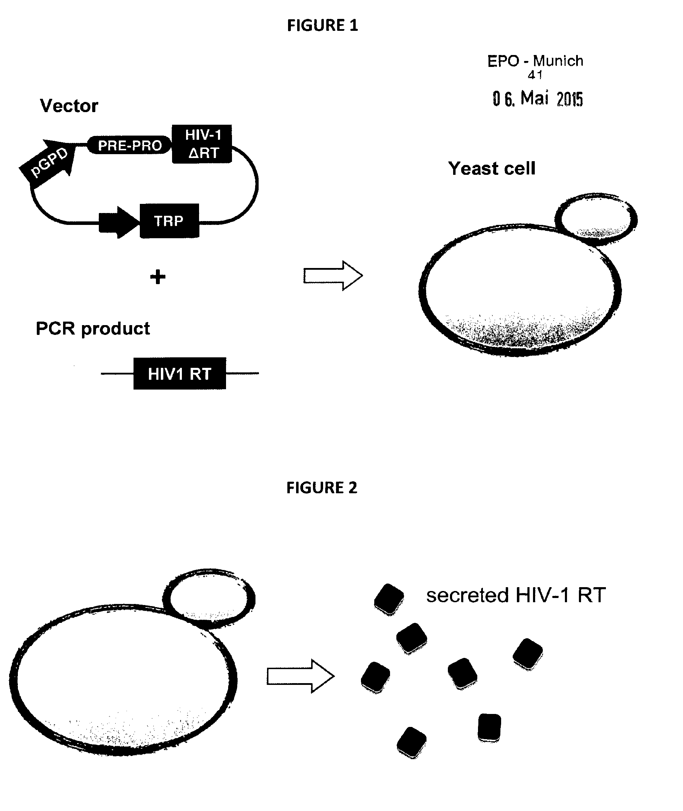

In the step a), the term "transforming" refers to the transfer of an exogenous nucleic acid molecule, preferably a double stranded DNA, into a host cell. Host cell containing such exogenous nucleic acid molecule or vector are called "transformed" or "recombinant" host cell. The transfer can occur by any means, such as by infection, conjugation, transformation, electroporation or microinjection. Methods of yeast cell transformation are well known in the art. Figure 1 illustrates the step a) of the method according to the invention.

The nucleic acid corresponds to a vector, such as a plasmid.

The term "vector" refers to extra chromosomal element often carrying genes which are not part of the central metabolism of the cell, and usually in the form of linear or circular double-stranded DNA molecules. Such elements may be autonomously replicating sequences, genome integrating sequences, phage or nucleotide sequences, linear or circular, derived from any source, in which a number of nucleotide sequences have been joined or recombined into a unique construction which is capable of introducing a promoter fragment and DNA sequence for a selected gene product along with appropriate 3' untranslated sequence into a cell. Vector may thus comprise an origin of replication functional in yeast. The term "origin of replication functional in yeast" refers to any nucleic acid sequence which allows replication of a vector or a plasmid independently from the chromosome. Generally, the origin of replication is functional in at least one or more of the following: *Saccharomyces cerevisiae, Candida albicans* and *C. maltosa, Hansenula polymorpha, Kluyveromyces fragilis and K. lactis, Pichia guillerimondii* and *P. pastoris, Schizosaccharomyces pombe,* and *Yarrowia lipolytica.* Suitable origins of replication include, for example, ars 1, centromere ori, and 2µ ori.

The promoters controlling the expression of RT used in the method of the invention are functional in yeast cells and are preferably constitutive. Examples of promoters functional in yeast that could be used in the present invention comprise, but are not limited to: the GAL1-10 promoter (SEQ ID NO: 15), the ADH1 promoter (SEQ ID NO: 16) and the strong GPD (Glyceraldehyde 3P DH) promoter (SEQ ID NO: 17). (MAYA et al., Biotechnol. Lett., vol.30, p:979-987, 2008). Preferably, the promoter used in the method of the invention is GPD.

As used herein, the term "signal peptide" refers to a sequence of about 20 amino acid residues found at the amino terminus of a nascent polypeptide chain. This signal peptide is necessary to address the polypeptide to the endoplasmic reticulum and sufficient to lead to the secretion of the aforementioned protein to the extracellular media. During the translocation in the Endoplasmic Reticulum and the Golgi, the signal peptide is cleaved and the protein is matured. The signal peptide allows the delivery in the culture media of complex mature proteins, and the potential sequences of such signal peptide are general knowledge of the skilled person. The signal peptide used in the method of the invention is functional in yeast. Preferably, the signal peptide is selected from the group comprising or consisting of pre-pro-MFα sequence of alpha mating factor (SEQ ID NO: 1), the pre-pro sequence of the alpha mating factor deleted of the MFα sequence (SEQ ID NO: 2), and the pre-pro sequence of K28 toxin (SEQ ID NO: 3).

Advantageously, the at least one nucleic acid encoding for HIV RT may also encode for a "positive selection marker" so as to select the transformed yeast cells. Such positive selection marker corresponds to a nucleic acid sequence whose expression allows for the survival of the transformed yeast cell under specific growth conditions, whereas the non transformed yeast cell is killed or does not grow under the same specific growth conditions. Preferred positive selection markers functional in yeast are survival genes, which include ADE2, HIS3, LEU2, ALG7, CUP1 or TRP1, the yeast cell to be transformed not comprising the chosen survival gene.

Preferably, said positive selection marker is the TRP1 gene (SEQ ID NO: 18), and is used with a transformed yeast cell, lacking a functional TRP1 gene in its genome. For selecting transformed yeast cell, the transformed yeast cell is cultured in a medium lacking tryptophan. Thus, only the transformed yeast cell expressing the TRP1 gene will be able to grow in this medium.

The step b) of culturing the transformed yeast cell is performed for a sufficient time so as to obtain correct production yield of the HIV RT protein in the culture medium. Such culturing step is done according to protocol well known from the skilled person. The transformed yeast cell is cultured at 30°C in shaking. By "a time sufficient" is meant a time comprised between 12 and 96 hours, preferably between 24 and 72hours, preferably 72h.

At the end of the culturing step b), the step c) of separating transformed yeast cell from culture medium so as to collect the supernatant is done by physical means well known from the man skilled in the art. Now, it is preferred to keep the transformed yeast cells alive during said separation step. Preferably, centrifugation is used for the separation of the transformed yeast cells from the supernatant. Typically, said centrifugation is done at 250 x g for 5min. The resulting pellet of transformed yeast cells is resuspended in culture media and further cultured at 30°C in shaking.

The supernatant contains the HIV RT mature enzyme encoded by the at least one nucleic acid present in the successfully transformed yeast cell (see figure 2). The enzymatic HIV RT activity is then assayed directly in the collected supernatant. Surprisingly, no purification step to recover HIV RT is needed. In fact, the presence in the supernatant of all metabolites resulting from the growth of the transformed yeast cell was naturally suspected to inhibit said RT activity, notably by degrading RNA templates.

By "assaying the activity of said HIV RT" in step d) is meant testing the ability of said enzyme to generate cDNA from a RNA template, which is preferably synthetic. Now, said HIV RT activity is assayed directly in the collected supernatant in step c). In fact, no further purification step is needed to recover HIV RT enabling the obtaining of a result in a shorter time and at lower costs.

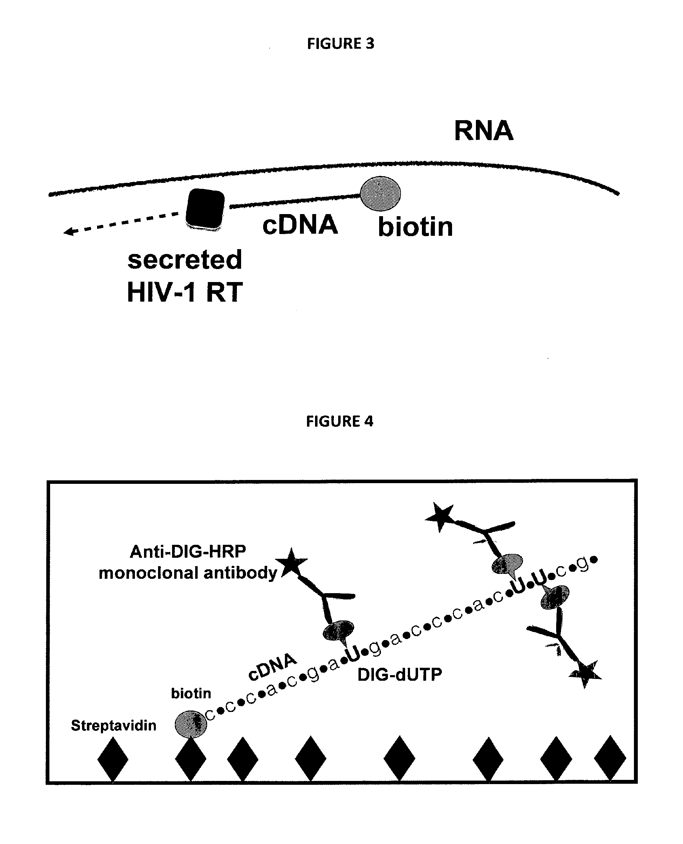

Said assay is preferably performed in the presence of the necessary reagents, including an RT initiation primer, such as poly T or specific primers, and free dNTPs. Preferably, said reagents further includes at list one modified dNTP such as dUTP, said modified dNTP including a marker such as digoxygenin. Still preferably, the RT initiation primer also comprises a marker so as to simplify the purification of the RT products. As an example, said marker is a biotinylated primer, such as a 5' biotinylated primer (see figure 3).

After the RT reaction has occurred, the obtained RT products are purified and then quantified. As an example, when they contain both biotinylated and digoxigenin labels, they are first purified using streptavidin (e.g. streptavidin coated plate), and then quantified using an anti-DIG antibody. Said antibody being coupled to an enzyme, such as peroxidase or alkaline phosphatase, its quantity, and thus the one of the RT products, can be determined by dosing said enzyme activity. As an example, for a peroxidase conjugated anti-DIG antibody to digoxigenin, the quantification step is done using a colorimetric substrate for this enzyme (see figure 4). The produced colored molecules are directly correlated to the RT products -i.e. newly synthetized cDNA-.

The RT activity is deduced from the amount of RT products, and thus the determination of said amount of RT products permits the determination of RT activity's variations.

The step e) of selecting a compound inhibiting HIV RT activity is done by selecting a compound, which can lower the RT enzymatic activity from at least 10%, at least 20%, at least 30%, at least 50%, at least 60%, at least 70%, at least 80%, and still preferably at least 90% as compared to the HIV RT activity obtained in the absence of any HIV RT inhibitor (such variation corresponds to a diminution of the amount of RT products from at least 10%, at least 20%, at least 30%, at least 50%, at least 60%, at least 70%, at least 80%, at least 90% respectively as compared to the amount of RT products obtained in the absence of said HIV RT inhibitors).

In a preferred embodiment, the invention provides a method for determining the susceptibility of a population of HIV viruses infecting a subject to a reverse transcriptase inhibitor. By "susceptibility", is intended a virus's response to a particular drug. A decreased or reduced susceptibility of a virus to a drug, or to a treatment using this drug, is indicative of a developing resistance mechanism by said virus. As an example, a mutation within HIV genome, and notably in Pol gene, can play a causal role in the reduced susceptibility to the anti-viral treatment. By contrast, a virus that has an increased or enhanced susceptibility to a drug is more vulnerable to the drug.

As used herein, the term "subject" denotes a human, male or female, infected by HIV virus.

Viral drug susceptibility is determined as the concentration of the compound, i.e. the antiviral agent, at which a given percentage of cDNA synthesis from synthetic RNA template is inhibited. As an example, the IC₅₀ value for a HIV RT inhibitor is the concentration at which 50% of the HIV RT activity is inhibited. The more efficient the HIV RT inhibitor is, the less newly synthetized cDNA from synthetic RNA is produced. A standard curve for drug susceptibility of the given HIV RT inhibitor can be determined by using varying concentrations of the reverse transcriptase inhibitor. Standard laboratory HIV isolates known to be either sensitive or resistant to said inhibitor can also be used as comparative references.

By the method of the invention, one can identify new compounds issued from bank compounds and having efficient inhibitory action on HIV RT.

Concerning the known HIV RT inhibitors, they are subdivided in three mains classes: the nucleoside RT inhibitors (NRTIs), the nucleotide RT inhibitors (NtRTIs) and the non-nucleoside RT inhibitors (NNRTIs).

NRTIs and NtRTIs share a similar mechanism of action. When reverse transcription occurs, HIV RT uses an NRTI or NtRTI triphosphate instead of natural nucleotide from the supply in the cell. Because these drugs have a slightly different structure than natural nucleotides, they cannot form the necessary chemical bonds, and natural nucleotides cannot be added on to continue the chain. Since HIV has no mechanism for correcting such mistakes, NRTIs and NtRTIs can interrupt reverse transcription and thereby halt HIV replication. Among all of the NRTIs and NtRTIs compounds currently used in therapeutic regimen, one can recite abacavir (ABC), didanosine (ddI), zalcitabine (ddC), lamivudine (3TC), emtricitabine (FTC), zidovudine (AZT) and stavudine (d4T) for NRTIs, and tenofovir (TFV) for NtRTIs.

Non-nucleoside Reverse Transcriptase Inhibitors (NNRTIs) interfere with reverse transcription by directly binding to the enzyme and retarding its function. These are usually small chemical molecules with a long half-life. Efavirenz (EFV), etravirine (ETV), nevirapine (NVP) and rilpivirine (RPV) are the NNRTIs currently used for the treatment of HIV seropositive patients.

In another preferred embodiment, the method of the invention is for determining the ability of the compound to inhibit the HIV RT expressed by different isolates of HIV viruses, wherein the successive steps a) to e) are repeated with distinct yeast transformed cells, each yeast transformed cell expressing the HIV RT of a distinct isolate of HIV virus.

In a particular embodiment, the method of the invention comprises a preliminary step of extracting HIV genome, i.e. viral RNA, from cell or cell-free body fluids issued from a subject infected by HIV-1 or HIV-2 virus. As an example, viral RNA, which binds specifically to a silica membrane, is washed to remove RNase and other contaminants before being eluted. Such methods are well known by the man skilled in the art and several commercial kits can be used for this purpose.

Since different isolates of HIV may infect a subject, the amplification of the reverse transcripts of the viral RNAs may lead to a pool of viral nucleic acid representative of the different isolates. A pool of nucleic acids from a subject may be obtained by gene amplification (Polymerase Chain Reaction technique, PCR) or by release of the reverse transcriptase by virtue of the action of restriction enzymes on purified viral DNA. For example, if the subject is a human patient infected by the HIV-1 or HIV-2 virus, the nucleic acid coding for HIV-1 or HIV-2 reverse transcriptase may be obtained from the lymphocytes or plasma of a whole blood sample obtained from said subject by well known methods (See for example, the methods disclosed in DWIGHT et al., Journal of Clinical Microbiology, vol.43, n11, pp5696-5704, 2005; and in SHAFER et al., Journal of Clinical Microbiology, vol.34, n7, pp1849-1853, 1996).

Following said viral RNA purification and on the basis of its general knowledge, the skilled person can simply design primers able for obtaining nucleic acid sequences encoding the HIV RT associated with said viral RNAs. Such obtaining is done by RT-PCR corresponding to a first reverse transcription of said viral RNAs, followed by the amplification of the obtained reverse transcription products by PCR.

As an example, the nucleic acid sequence coding for the HIV-1 RT may be amplified by RT-PCR from a blood sample of an infected patient using the pair of primers defined by the nucleic acid sequences SEQ ID NO: 21 and SEQ ID NO: 22. This amplification results in the obtaining of a 1512 base pair long template which encodes for the first two-third of the HIV-1 RT (i.e. 75% of the total length of the coding sequence of HIV-1 RT starting from 5'). It is well established that all the resistance mutations occurred within this portion of HIV-1 RT.

The nucleic acid encoding for the last one-third of HIV-1 RT is already within the vector.

Advantageously, a nested PCR is done so as to increase sensitivity. Such nested PCR is done on the RT-PCR products of a first round of PCR amplification -e.g. the RT-PCR DNA fragment as obtained after an amplification using the pair of primers of SEQ ID NO: 21 and SEQ ID NO: 22- by a second round of PCR amplification using a second pair of primers. As an example, such a second round of PCR amplification can be performed using the pair of primers defined by the nucleic acid sequences SEQ ID NO: 4 and SEQ ID NO: 5 resulting in a 1316 base pair long PCR end-product.

From the nested PCR product, RT sequence is amplified using the pair of primers of SEQ ID NO: 29 and SEQ ID NO: 5 which PCR end-product is 933 base pair long while the amplification product using the pair of primers of SEQ ID NO: 6 and SEQ ID NO: 7 is 850 base pair long. Now the exact PCR products length is dependent on the HIV type, and the subtypes (for HIV-1) infecting the patient. Preferably, the sequence of the at least one nucleic acid encoding for HIV-1 RT is selected from the group comprising or consisting of SEQ ID NO: 12 and SEQ ID NO: 8, wherein SEQ ID NO: 12 is a consensus sequence of HIV-1 Pol and SEQ ID NO: 8 is a consensus sequence of HIV-1 RT.

As an example, the nucleic acid sequence coding for the HIV-2 RT may be amplified by RT-PCR from a blood sample of an infected patient using the pair of primers defined by the nucleic acid sequences SEQ ID NO: 27 and SEQ ID NO: 28. This amplification results in the obtaining of a 1317 base pair long template. Preferably, the sequence of the at least one nucleic acid encoding for HIV-2 RT is selected from the group comprising SEQ ID NO: 14.

Finally, the pool of nucleic acids sequences coding for HIV-RT obtained by the amplification of the viral RNAs from an infected subject is representative of the HIV isolates infecting such subject.

In still another preferred embodiment, the method of the invention is for determining an efficient therapeutic regimen that can be used for treating a subject suffering from HIV infection, wherein the successive steps d) and e) are repeated with each compound to be tested, and wherein:
i) said each compound to be tested are selected from the group comprising HIV RT inhibitors, and
ii) the transformed yeast cell corresponds to a pool of transformed yeast cells, which pool of transformed yeast cells has been transformed by a pool of nucleic acids encoding for the pool of HIV RT expressed by the HIV viruses infecting said subject.

Preferably, the pool of nucleic acids encoding for HIV RT are comprised within a pool of vectors.

In a particular embodiment, such vectors are obtained by homologous recombination following the step of transforming yeast cells by both i) a pool of nucleic acids obtained from the viral RNAs of an infected subject and ii) a vector comprising a specific homologous recombination site so as to obtain a pool of vectors comprising nucleic acids encoding for the HIV RT expressed by the pool of HIV viruses infecting said subject.

The term "homologous recombination site" refers to a site that allows the introduction of the nucleic acid fragment of interest into a vector or a shuttle vector by homologous recombination. Homologous recombination is, briefly, the process of strand exchange that can occur spontaneously with the alignment of homologous sequences (i.e. sets of complementary strands). As is known in the art, yeasts are efficient at homologous recombination (see for example ORR-WEAVER et al., Proc. Natl. Acad. Sci. USA, vol.78, pp6345-6358, 1981; MA et al., Gene, vol.58, pp201-216, 1987; Petermann, Nucleic Acids Res., vol.26, n°9, pp:2252-2253, 1998; each incorporated herein by reference). Methods and conditions allowing homologous recombination are well known in the art.

Thus, in general, the homologous recombination site contains two distinct, but generally contiguous, regions. The first region, referred to herein as the 5' region, is generally identical to the 5' region flanking the nucleic acid fragment of interest to insert into the vector. The second region, referred to herein as the 3' region, is generally identical to the 3' region flanking the nucleic acid fragment of interest to insert into the vector. Preferably, the 5' and 3' regions are each at least 12 or 15 nucleic acids long. More preferably, the 5' and 3' regions are each at least about 20 or 30 nucleic acids long, and more preferably at least about 50 nucleic acids long, and most preferably about 60 nucleic acids long. According to the invention, the homologous recombination site sequence refers to any nucleic acid sequence which is unique to the vector in that the vector does not comprise another sequence corresponding to the sequence of the homologous recombination site and which is homologous with the flanking regions of the nucleic acid fragment of interest to insert.

The nucleic acid sequence encoding for a HIV RT comprises the same homologous 5' and 3' recombination regions as those contained within the vector used to transform yeast cells. The nucleic acid sequence encoding for a HIV RT is thus the "nucleic acid fragment of interest" that is inserted into the vector at the homologous recombination site. When homologous recombination occurs, the nucleic acid fragment encoding for a HIV RT is inserted in the vector. The 5' and 3' recombination regions flanking the nucleic acid encoding for a HIV RT replace the 5' and 3' recombination regions of the recombination site during homologous recombination.

So as to limit backgrounds corresponding to transformed yeast cells comprising vectors not having integrated by homologous recombination any nucleic acid fragment of interest so as to obtain a nucleic acid encoding for HIV RT, said vector may further comprises a negative selection marker within the homologous recombination site.

A "negative selection marker" is a nucleic acid sequence that kills, prevents growth of or otherwise selects against cells expressing said negative selection marker usually upon application of an appropriate exogenous agent. An example of a negative selection gene is the "URA" (orotidine 5' phosphate decarboxylase) gene and more preferably a "URA3". URA3 gene as used herein includes a URA3 gene derived from any yeast, preferably from *Saccharomyces cerevisiae* (SEQ ID NO: 23) or *Kluyveromyces lactis.* URA3 is able to convert the 5-Fluoroorotic acid (5-FOA) into a toxic compound 5-fluorouracil causing cell death.

This negative selection marker, such as URA3, is located between the two distinct regions of the recombination site and is maintained in the vector only if no homologous recombination occurred. This negative selection marker thus enable not to select transformed yeast cells comprising vector not having integrated any nucleic acid fragment of interest by homologous recombination. For an URA3 negative selection marker, such "selection" is obtained by adding 5-FOA to the culture medium.

This technology of transformed yeast cells obtained by homologous recombination is detailed in the international patent application WO 2012/176016 A1, which teaching concerning is incorporated herein by reference.

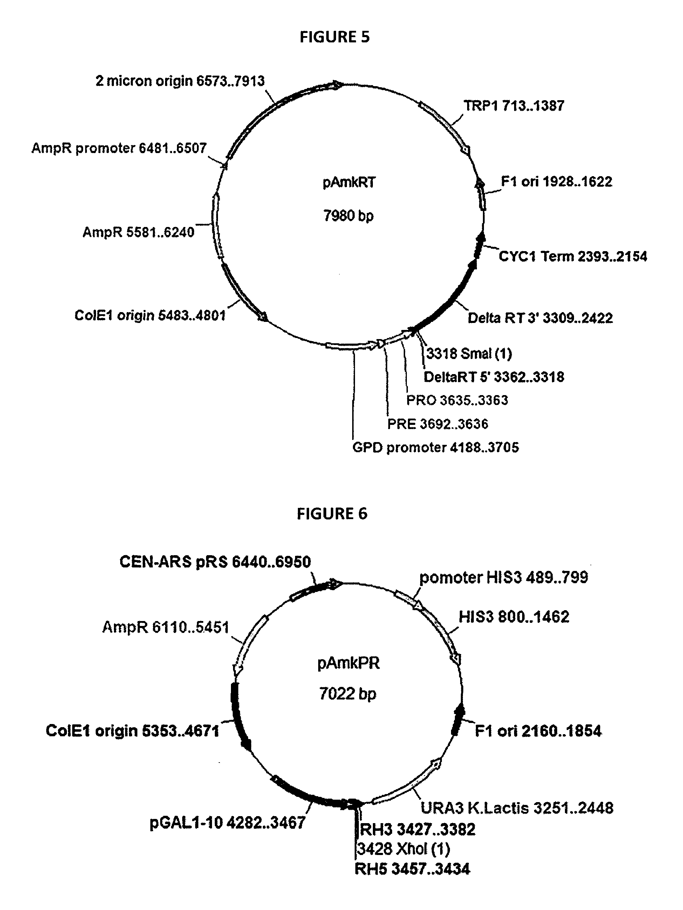

As an example, and for the obtaining by homologous recombination of a pool of vectors comprising nucleic acids encoding for the HIV-1 RT expressed by the pool of HIV-1 viruses infecting said subject, the pair of primers of SEQ ID NO: 6 and SEQ ID NO: 7 or the pair of primers of SEQ ID NO:29 and SEQ ID NO: 5 can be used so as to obtain a pool of nucleic acid fragments, which fragments are then integrated by homologous recombination within the vector pAmkRT (SEQ ID NO: 26) at position 2448-3427. The map of pAmkRT vector is detailed in figure 5.

As an example, and for the obtaining by homologous recombination of a pool of vectors comprising nucleic acids encoding for the HIV-2 RT expressed by the pool of HIV-2 viruses infecting said subject, the pair of primers of SEQ ID NO: 27 and SEQ ID NO: 28 can be used so as to obtain a pool of nucleic acid fragments, which fragments are then integrated by homologous recombination within the vector pAmkRT (SEQ ID NO: 26).

In a second object, the method of the invention as described previously is for further identifying a compound inhibiting HIV protease (HIV PR), wherein:
- the step a) further comprises the transforming of said yeast cell by at least one nucleic acid encoding for a HIV PR, wherein the nucleic acid sequence encoding for HIV PR is under the control of a promoter;
- the step b) is for obtaining enough transformed yeast cells for testing HIV PR activity, and
- the step c) of separating the cultured transformed yeast cells from the supernatant is also for harvesting the cultured transformed yeast cell of step b); and
wherein said method further comprises the steps:
f) Assaying the activity of said HIV PR in the cultured transformed yeast cells in the presence or absence of a compound; and
g) Selecting a compound inhibiting HIV PR activity in step f).

The term "HIV protease" refers to a protein functions to cleave the large viral precursor proteins into individual enzymes and structural proteins, which produces infectious viral particles. The HIV-1 protease (PR) is a virus-encoded proteolytic enzyme that is initially synthesized as part of the GagPol polyprotein. PR autoprocessing is a regulated process by which the precursor PR catalyzes the cleavage reactions leading to liberation of the free mature PR upon or shortly after progeny virion is released from the infected cell. HIV-1 PR is an aspartic protease composed of 99 residues. The released mature PR recognizes and cleaves at least ten sites in the Gag and GagPol polyproteins Accurate and precise PR processing of these sites is absolutely required for the production of infectious progeny virion.

The nucleic acid sequence encoding for a HIV PR may be obtained from a biological sample as defined previously. Preferably, the biological sample used for obtaining the nucleic acids encoding for HIV PR and for HIV RT is the same.

In the step a) of the method of the invention, the yeast cell previously transformed by at least one nucleic acid encoding for HIV RT, is simultaneously transformed by at least one nucleic acid encoding for a HIV PR.

Advantageously, the at least one nucleic acid encoding for HIV PR may also encode for a "positive selection marker" so as to select the transformed yeast cells like for the at least one nucleic acid encoding for HIV RT. When the at least one nucleic acid encoding for HIV PR is distinct from the at least one nucleic acid encoding for HIV RT, two distinct positive selection markers may be used, while a single positive selection marker may be used when the nucleic acid sequences encoding for both HIV RT and HIV PR are comprised in a single nucleic acid molecule.

As compared to the at least one nucleic acid encoding for RT, the at least one nucleic acid encoding for PR is preferably under the control of an inducible promoter. By "inducible promoter" is intended a promoter that requires to be activated to activate the transcription of the gene located upstream. Such system allows a fine regulation of the expression of genes. Within the group of inducible promoter, there are promoters modulated by abiotic factors (i.e., light, oxygen levels, heat, cold, etc...) or chemical compounds, not found naturally in the transformed yeast cell (i.e., antibiotics, copper, alcohol, steroids, etc...) or not synthetized by the transformed yeast cell (i.e., amino acids, carbohydrates, etc...). Preferably, the inducible promoter used in the method of the invention is GAL1-10 promoter (SEQ ID NO: 15).

As compared to the method disclosed previously, the supernatant will be now used for testing HIV RT activity, while the transformed yeast cells will used for testing the HIV PR activity.

By « assaying » the HIV PR activity is meant testing the ability of compound to inhibit HIV PR activity, which compound may be selected from a group comprising the molecules of a library, chemical molecules, natural molecules and molecules extracted from plants. Now, the compound may be also known as having a HIV PR inhibition activity. In this hypothesis, the method of the invention may have different purposes: i) determining the IC₅₀ of the compound, ii) determining the scope of the protease inhibition activity on different HIV genotypes and/or subtypes and/or isolates, and/or determining an efficient therapeutic regimen for an infected subject. In this later case, it is intended that the therapeutic regimen can also include HIV RT inhibitors identified by the HIV RT assay performed in the supernatant of the transformed yeast cells. The compound tested on the PR activity may be the same or not than the one tested on the RT activity, preferably a distinct compound.

Since the expression of the HIV PR in yeast cells induced their death, the nucleic acid encoding for HIV PR used for transforming yeast cells is under the control of an inducible promoter, such as GAL1 (*See.* international patent application WO 2011/007244). By inhibiting the HIV PR activity, the PR inhibitors thus protect the transformed yeast cells from dying and allow them to grow.

In a preferred embodiment, the invention provides a method for determining the susceptibility of a population of HIV infecting a subject to a protease inhibitor.

Viral drug susceptibility is determined as the concentration of the compound, i.e. the antiviral agent, at which a given percentage of transformed yeast cells survived. As an example, the IC₅₀ value for a HIV PR inhibitor is the concentration allowing the viral protease expressing cells to grow 50% less than cells not expressing the viral protein. The more efficient the HIV PR inhibitor is, the more the transformed yeast cells survive and then grow. A standard curve for drug susceptibility of the given HIV PR inhibitor can be determined by using varying concentrations of the protease inhibitor. Standard laboratory HIV isolates known to be either sensitive or resistant to said inhibitor can also be used as comparative references.

By the method of the invention, one can identify new compound issued form bank compounds and having inhibitory action of HIV PR.

Now, the method of the invention may also be used on known HIV PR inhibitors, which are noncleavable substrate analogs targeting the catalytic site of the mature PR with high binding affinities. As an example of such HIV PR inhibitors, one can cite amprenavir (Agenerase®), atazanavir (Reyataz®), darunavir (Prezista®), fosamprenavir (Telzir®, Lexiva®), indinavir (Crixivan®), lopinavir/ritonavir (Kaletra®, Aluvia®), nelfinavir (Viracept®), ritonavir (Norvir®), saquinavir (Invirase®) and tipranavir (Aptivus®). Most of the HIV PR inhibitors are peptidomimetics. Second-generation protease inhibitors (atazanavir, darunavir, fosamprenavir, lopinavir, and tipranavir) efficient against HIV variants that have developed resistance to older drugs are in this class. Darunavir and tipranavir differ from the others in their structure since they are synthetic non-peptidic drugs.

As previously disclosed, different isolates of HIV may infect a subject. Thus, the amplification of the reverse transcripts of the viral RNAs may lead to a pool of viral nucleic acid representative of such different isolates. A pool of nucleic acid sequences coding for HIV PR from a subject may be obtained by gene amplification (Polymerase Chain Reaction technique, PCR) or eventually by release of the protease by virtue of action of restriction enzymes on purified viral DNA.

Again, the skilled person can simply design on the basis of its general knowledge primers able to amplify nucleic acid sequences encoding the HIV PR associated with the viral RNAs obtained from a biological sample of a an infected subject.

As an example, the nucleic acid sequence coding for the HIV PR may be amplified by RT-PCR from a blood sample of an infected subject using the pair of primers defined by the nucleic acid sequences SEQ ID NO: 21 and SEQ ID NO: 22. This specific amplification results in the obtaining of a 1512 base pair long template, which is identical to the one previously described for the obtaining of a nucleic acid encoding for a HIV RT.

Then, a nested PCR can be done by amplifying a fragment in the previously obtained RT-PCR product used as a template. As an example, this second PCR can be performed by using the pair of primers defined by the nucleic acid sequences SEQ ID NO: 4 and SEQ ID NO: 5 resulting in a 1316 base pair long PCR end-product.

From the nested PCR product, PR sequence is amplified using the pair of primers of SEQ ID NO: 9 and SEQ ID NO: 10 or the pair of primers of SEQ ID NO: 9 and SEQ ID NO: 11. For such a specific amplification using the pair of primers of SEQ ID NO: 9 and SEQ ID NO: 10, the obtained PCR end-product is 417 base pair long while the amplification product using the pair of primers of SEQ ID NO: 9 and SEQ ID NO: 11 is 411 base pair long. The amplification products are precursor of HIV PR. Now, and just like for HIV RT, the exact PCR products length depends upon the HIV type and subtypes (for HIV-1) infecting the subject. Preferably the sequence of the at least one nucleic acid encoding for HIV PR is selected from the group comprising or consisting of SEQ ID NO: 12 and SEQ ID NO:13, wherein SEQ ID NO: 12 is a consensus sequence of HIV-1 Pol and SEQ ID NO: 13 is a consensus sequence of HIV-1 PR.

Again, the pool of nucleic acids sequences coding for HIV-PR obtained by the amplification of the viral RNAs from an infected subject is representative of the HIV isolates infecting said subject, just like for the HIV-RT mentioned previously.

When the method of the invention is for determining an efficient therapeutic regimen that can be used for treating a subject suffering from a HIV infection, the successive steps d) to g) are repeated with each compound to be tested, and wherein:
i) said each compound to be tested are selected from the group comprising HIV RT inhibitors and HIV PR inhibitors, and
ii) the transformed yeast cell corresponds to a pool of transformed yeast cells, which pool of transformed yeast cells has been transformed by a pool of nucleic acids encoding for the pool of HIV RT and HIV PR expressed by the HIV viruses infecting said subject.

Preferably, the pool of nucleic acids encoding for HIV RT and HIV PR are comprised within a pool of vectors.

In a particular embodiment, such vectors are obtained by homologous recombination following the step of transforming yeast cells by
- a pool of nucleic acids obtained from the viral RNAs of an infected subject,
- a vector comprising a specific homologous recombination site so as to obtain a pool of vectors comprising nucleic acids encoding for the HIV RT expressed by the pool of HIV viruses infecting said subject, and
- a vector comprising a specific homologous recombination site so as to obtain a pool of vectors comprising nucleic acids encoding for the HIV PR expressed by the pool of HIV viruses infecting said subject.

The vectors comprising specific homologous recombination sites so as to obtain a pool of vectors comprising nucleic acids encoding respectively for the HIV RT and HIV PR expressed by the pool of HIV viruses infecting said subject may be distinct or not.

When such vectors are distinct, they preferably comprised distinct positive selection markers and, eventually, distinct negative selection markers.

As previously mentioned, preferred positive selection markers functional in yeast are survival genes, which include ADE2, HIS3, LEU2, ALG7, CUP1 or TRP1, the yeast cell to be transformed not comprising the chosen survival gene. When vectors are distinct, preferred positive selection gene is HIS3.

As previously mentioned, an example of a negative selection gene is the "URA" (orotidine 5' phosphate decarboxylase) gene and more preferably a "URA3". URA3 gene as used herein includes a URA3 gene derived from any yeast, preferably from *Saccharomyces cerevisiae* (SEQ ID NO: 23) or *Kluyveromyces lactis* (SEQ ID NO: 24).

As an example, and for the obtaining by homologous recombination of a pool of vectors comprising nucleic acids encoding for the HIV-1 PR expressed by the pool of HIV-1 viruses infecting said subject, the pair of primers SEQ ID NO: 9 and SEQ ID NO: 10 or SEQ ID NO: 9 and SEQ ID NO: 11, can be used so as to obtain a pool of nucleic acid fragments, which fragments are then integrated by homologous recombination within the vector pAmkPR (SEQ ID NO: 25) at position 3382-3457. The map of pAmkPR vector is detailed in figure 6.

As an example, and for the obtaining by homologous recombination of a pool of vectors comprising nucleic acids encoding for the HIV-2 PR expressed by the pool of HIV-2 viruses infecting said subject, the pair of primers of SEQ ID NO: 30 and SEQ ID NO: 31 can be used so as to obtain a pool of nucleic acid fragments, which fragments are then integrated by homologous recombination within the vector pAmkPR.

Preferably, the sequence of the at least one nucleic acid encoding for HIV-2 PR is selected from the group comprising SEQ ID NO:32.

Thus, an advantage of the method of the invention is to identify both HIV RT and HIV PR inhibitors from a single assay, the HIV RT being tested directly in the supernatant of the transformed yeast cell and the HIV PR being tested in the transformed yeast cell. By using the method of the invention, the clinician is able to rapidly adapt the therapeutic regimen of a patient infected by HIV.

Still another object of the invention is directed to a kit for identifying a compound inhibiting HIV RT, wherein said kit comprises:
(i) a yeast cell suitable for transformation,
(ii) a set of primers, and a vector, so as to obtain a nucleic acid encoding for a HIV RT and a peptide signal useful in the method for identifying a compound inhibiting HIV RT as described previously.

As used herein, the term "kit" refers to any delivery system for delivering materials. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contains a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

The present kit can also include one or more reagents, buffers, hybridization media, nucleic acids, primers, nucleotides, probes, molecular weight markers, enzymes, solid supports, databases, computer programs for calculating dispensation orders and/or disposable lab equipment, such as multi-well plates, in order to readily facilitate implementation of the present methods. Enzymes that can be included in the present kit include nucleotide polymerases and the like. Solid supports can include beads and the like whereas molecular weight markers can include conjugable markers, for example biotin and streptavidin or the like.

Advantageously, the kit is made up of instructions for carrying out the method described herein. The instructions can be provided in any intelligible form through a tangible medium, such as printed on paper, computer readable media, or the like.

According to a preferred embodiment, the kit comprises i) at least one pair of primers enabling the obtaining of a pool of nucleic acids by amplification (e.g. by RT-PCR) from the viral RNAs of an infected subject and ii) a vector comprising a specific homologous recombination site so as to obtain a pool of vectors comprising nucleic acids encoding for the HIV RT expressed by the pool of HIV viruses infecting said subject by homologous recombination with the pool of nucleic acids.

Preferably, said i) at least one pair of primers is SEQ ID NO: 29 and SEQ ID NO: 5 or SEQ ID NO: 6 and SEQ ID NO: 7, and said ii) vector is pAmkRT (SEQ ID NO: 26).

In a more preferred embodiment, the kit of the invention further comprises iii) a set of primers, and a vector, so as to obtain a nucleic acid encoding for a HIV PR useful in the method for identifying a compound inhibiting HIV PR, wherein the sets of primers in ii) and iii) being identical or distinct.

According to this preferred embodiment, the kit comprises i) at least one pair of primers enabling the obtaining of a pool of nucleic acids by amplification (e.g. by RT-PCR) from the viral RNAs of an infected subject and ii) a vector comprising a specific homologous recombination site so as to obtain a pool of vectors comprising nucleic acids encoding for the HIV PR expressed by the pool of HIV viruses infecting said subject by homologous recombination with the pool of nucleic acids.

Preferably, said iii) at least one pair of primers is SEQ ID NO: 9 and SEQ ID NO: 11, or SEQ ID NO: 9 and SEQ ID NO: 10 and said iv) vector is pAmkPR (SEQ ID NO: 25).

In a preferred embodiment, said kit further comprises a culture medium deficient for at least one amino acid residue, notably for tryptophan and histidine.

The following examples are provided herein for purposes of illustration only and are not intended to be limiting unless otherwise specified.

### EXAMPLES

### 1 - Viral RNA purification from human plasma

400µl of plasma sample is treated for RNA purification using any marketed kit (Qiagen EZ1 Virus Mini Kit for example) following the manufacturer instructions.
Total purified viral RNA was harvested in 60 µl of TE (TrisHCl 10mM pH8.0, EDTA0.5mM).

### 2 - Amplification, by RT-PCR, of a viral DNA fragment (nt 1573 to nt 3085)

A viral DNA fragment (nt 1573 to nt 3085) was amplified from purified RNA as follows:
- 5 µl of extracted viral RNA was mixed with,
- primer of SEQ ID NO: 21 at 10µM final concentration
- primer of SEQ ID NO: 22 at 10µM final concentration
- reaction mix containing 0.4mM of each dNTPs and 2.4mM MgSO4 (from life technologies - 10928-042)
- 0.5 of RT/Platinum Taq mix from Life technologies (ref N° 10928-042) in a final 25 µl reaction volume.

If the viral load was lower than 500 copies/ml, the volume of the extracted viral RNA, the RT/Platinum Taq mix (Life technologies ref N° 10928-042), and the total reaction was multiplied by a factor of 2.

The 1512 nt length produced viral cDNA was further amplified by performing the following polymerization procedure:
- 30 min at 45 °C
- 2 min at 94 °C
   - followed by 40 cycles of:
- 15 sec at 94°C
- 20 sec at 56 °C
- 2 min at 72°C
   - and ended with 10 min at 72°C.

### 3 - Second round of amplification of a viral DNA fragment (nt 1692 to nt 3008)

A viral DNA fragment (nt 1692 to nt 3008) harboring the viral DNA was amplified from 1µl of RT-PCR product with:
- primer of SEQ ID NO: 4 at 10µM final concentration
- primer of SEQ ID NO: 5 at 10µM final concentration
- 1x PCR buffer with MgCl2 (from ROCHE ref N°12161567001)
- 2 units of FastStart Taq DNA polymerase (from ROCHE ref N° 12032953001) in a final 50µl reaction volume.

The 1316 nt length produced viral cDNA was further amplified by performing the following polymerization procedure:
- 4 min at 95 °C
   - followed by 35 cycles of:
- 30 sec at 94 °C
- 45 sec at 56 °C
- 2 min at 72 °C
   - ended with 10 min at 72 °C.

### 4 - Amplification, by PCR, of a viral DNA fragment (nt 1729 to nt 2140) harboring the viral Protease gene

A viral DNA fragment (nt 1729 to nt 2140) harboring the viral Protease gene was amplified from 1µl of PRO1-RT20 PCR product with:
- primer of SEQ ID NO: 9 at 10µM final concentration
- primer of SEQ ID NO:11 at 10µM final concentration
- 1x PCR buffer with MgCl2 (from ROCHE ref N°12161567001)
- 2 units of FastStart Taq DNA polymerase (from ROCHE ref N° 12032953001) in a final 50µl reaction volume.

The 411 nt length produced viral cDNA was further amplified by performing the following polymerization procedure:
- 4 min at 95 °C
   - followed by 40 cycles of:
- 30 sec at 94 °C
- 30 sec at 55 °C
- 1 min at 72 °C
   - ended with 7 min at 72 °C.

5µl of the reaction was then analyzed on an agarose gel (1.5 % agar) for size determination.

Total amplified DNA was further purified on Qiagen Qiaquick PCR purification kit following manufacture instructions, and stored in 50µl of TE buffer.

### 5 - Amplification, by PCR, of a viral DNA fragment (nt 2095 to nt 2945) harboring the viral Reverse Transcriptase gene

A viral DNA fragment (nt 2095 to nt 2945) harboring the viral Reverse Transcriptase gene was amplified from 1µl of the previously produced PRO1-RT20 DNA fragment with:
- primer of SEQ ID NO: 6 at 10µM final concentration
- primer of SEQ ID NO: 7 at 10µM final concentration
- 1x PCR buffer with MgCl2 (from ROCHE ref N°12161567001)
- 2 units of FastStart Taq DNA polymerase (from ROCHE ref N°12032953001) in a final 50µl reaction volume.

The 850 nt length produced viral cDNA was further amplified by performing the following polymerization procedure:
- 4 min at 95 °C
   - followed by 40 cycles of:
- 30 sec at 94 °C
- 30 sec at 55 °C
- 1 min at 72 °C
   - ended with 7 min at 72 °C.

If the viral load was lower than 500 copies/ml, the volume of the RT-PCR product was multiplied by a factor of 10.

5µl of the reaction was then analyzed on an agarose gel (1.5 % agar) for size determination.

Total amplified DNA was further purified on Qiagen Qiaquick PCR purification kit following manufacture instructions, and stored in 50µl of TE buffer.

### 6 - Yeast cell transformation (according to "Transformation of yeast by lithium acetate/single stranded carrier DNA/polyethylene glycol method. Gietz RD, Woods RA. Methods Enzymol.;vol350: pp87-96, 2002.)

YAB22 (HIS-, TRP-) cells were grown in 50ml YPD to exponential phase.

When the cell suspension achieved an OD600nm between 0,5 and 0,8, ∼3x10⁷ cells (assuming that 10⁷ cells/ml = 1 OD at 600nm) were harvested by centrifugation at 1300xg for 5 min. Cells were then washed twice with sterile water followed by a wash in 3 ml of Lithium Acetate 100mM in TrisHCl 10mM pH7.5 and EDTA 1mM pH8.0. The supernatant was discarded and the cells were resuspended in 600µl of the same solution.

50 µl of the cell suspension were incubated with:
- 0,2 µg of dephosphorylated XhoI single cut vector pAmkPR (for viral protease expression in the cell), and/or,
- 0,2 µg of dephosphorylated SmaI single cut vector pAmkRT (for viral reverse transcriptase secretion into the cell medium), and
- 5µg salmon sperm DNA, and
- 250 mg of Poly-ethylene glycol (3350MW) to a final 42%, and
- 25 µl of viral Protease gene DNA PCR product, and/or
- 25 µl of viral Reverse Transcriptase gene DNA PCR product
in a final 600 µl volume reaction.

After 30 min incubation at 30°C, DMSO was added to 3.33 % final. A heat shock at 42°C for 15 min was then applied to the cells followed by cell pelleting at 1300xg for 3 min. The supernatant was discarded and selection for cells transformed with one or both plasmids (harboring the Protease and the Reverse Transcriptase genes) were performed in 3 ml synthetic complemented medium without HIS for PR expression vector selection and synthetic complemented medium without TRP for RT expression vector selection. Transformed cells are incubated 3 days at 30°C with shacking.

### 7 - Protease Resistance Analysis

Transformed cells (that grew in HIS lacking media) were washed twice in sterile water and then resuspended in 7 ml SGalRC-HIS medium (composed by 1.7g/L yeast nitrogen without amino acids, 5g/L Ammonium Sulfate, 0.77g/L amino acids mixture without histidine, and 20g/L Galactose) to a final cell density corresponding to 0.15 OD600nm. Aliquots of 60 µl of the cell suspension were distributed per well.

Eight serial one to one dilutions of Protease inhibitors in SGalRC-HIS medium were performed starting at the following concentrations:

| **Protease inhibitors** | **Final concentration (µM)** |
|---|---|
| Amprenavir (APV) | 200 |
| Atazanavir (ATV) | 100 |
| Darunavir (DRV) | 20 |
| Indinavir (IDV) | 100 |
| Lopinavir (LPV) | 50 |
| Nelfinavir (NFV) | 25 |
| Ritonavir (RTV) | 20 |
| Saquinavir (SQV) | 400 |
| Tipranavir (TPV) | 10 |

120µl of each Protease Inhibitor concentration was added to an individual well (one well=one inhibitor at a specific concentration), diluting the Inhibitor concentration by one third.

After 48 hours at 30°C, cell density in each well was measured at OD600nm.

IC50 value for each inhibitor was defined as the inhibitor concentration allowing the viral protease expressing cells to grow 50% less than cells not expressing the viral protein.

### 8 - Reverse Transcriptase Resistance Analysis

Transformed cells (that grew in TRP lacking media) were incubated for 16h with shacking in 13 mL rich medium (Yeast extract Peptone Dextrose) at a final cell density 0,06 OD / mL.

13 mL cell culture supernatant was collected after centrifugation at 1500 rpm for 5min and kept as the source for *in vitro* Reverse Transcriptase resistance analysis.

HIV Reverse Transcriptase resistance to reverse transcriptase inhibitors was analyzed as follows.

In a final volume of 150µl, 120µl of transformed cell's medium were incubated in 56 mM Tris-HCl, 56 mM KCl and 9 mM MgCl2 with:
- 1.76µg of MS2 RNA (SEQ ID NO: 20), and
- 0.059 pmoles of 5'biotinylated primer of SEQ ID NO: 19, and
- dNTP mixture (1.5pmoles of each one; dATP,, dGTP, dCTP, and dTTP), and
- 0.19 pmoles of DIG (digoxigenin) modified dUTP, and
- 30 µl of one of each concentration out of eight serial one to one dilutions of Reverse Transcriptase inhibitors in water (in this way the inhibitor was further diluted 1:3).

The Reverse Transcriptase inhibitor's one to one dilution started at the following concentrations:

| **RT inhibitors** | | **Final concentrations (µM)** |
|---|---|---|
| **Classes** | **Name** | - |
| **NNRTI** | Efavirenz (EFV) | 0.05 |
| | Etravirine (ETV) | 0.1 |
| | Nevirapine (NVP) | 20 |
| | Rilpivirine (RPV) | 100 |
| **NtRTI** | Tenofovir (TFV) di phosphate | 100 |
| **NRTI** | Didanosine (ddI) tri-phosphate | 100 |
| | Zalcitabine (ddC) tri-phosphate | 100 |
| | Lamivudine (3TC) tri-phosphate | 100 |
| | Emtricitabine (FTC) tri-phosphate | 100 |
| | Carbovir (CBV) tri-phosphate | 100 |
| | Zidovudine (AZT) tri-phosphate | 100 |
| | Stavudine (d4T) tri-phosphate | 100 |

The mixture reaction was incubated at 37°C for an overnight period (16 - 20 hours).

At the end of the incubation, each reaction mixture was transferred to a streptavidin coated flat bottom well for ELISA based RNA retro-transcribed DNA determination.

Wells were washed three times with 200µl of wash buffer (0.1%BSA and 0.05% Tween in PBS) before 30min incubation at room temperature with 100µl of a 1:1000 dilution of Peroxidase -conjugated monoclonal mouse anti-DIG (from Jackson Immunosearch ref N°JIR 200-032-156) in wash buffer.

After 5 washes with 200µl wash buffer, 100 µl of Ultra TMB peroxidase substrate (from Perbio Scientific ref 34024) were added for a 5 to 30 minutes peroxidase reaction. The reaction was stopped by adding 100µl of 2M phosphoric acid.

The amount of produced yellow colored molecule, representing the amount of newly synthetized cDNA, was measured by absorbance at 405 nm.

IC50 value for each inhibitor was defined as the inhibitor concentration inhibiting by half the viral reverse transcriptase activity.

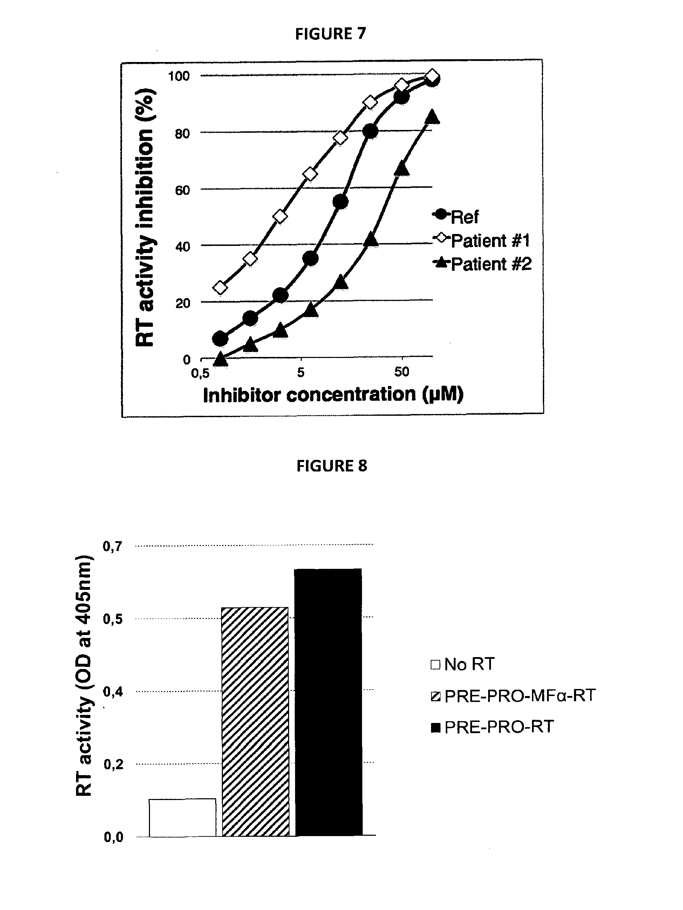

As shown in figure 7, IC50 value for HIV RT inhibitor can be determined both for a reverse transcriptase reference from a well defined HIV-1 or HIV-2 isolate (Ref.) and for a reverse transcriptase resulting from HIV infected patients (Patient #). Both RT Ref. and RT from Patients are incubated with increasing concentrations of RT inhibitor. Ref. is already known to be sensitive to the RT inhibitor. IC50 values are defined as the inhibitor concentrations for which reverse transcriptase activity is reduced by half. A lower IC50 value for patient #1 compared to IC50 value of Ref. is indicative of a higher sensitivity to the tested RT inhibitor. By contrast, a higher IC50 value for patient #2 compared to IC50 value of Ref. is indicative of a loss of sensitivity (i.e. of emerging resistant isolate) of an infecting virus to the tested RT inhibitor.

### RESULTS

### 1 - Impact of the signal peptide on HIV RT secretion

Fusions of HIV RT coding sequence to the prepro segment of prepro-alpha-factor have been used to secrete heterologous proteins from yeast. The fusions contain the amino half of prepro-alpha-factor, including the segments needed for proteolytic excision of the protein of interest, -i.e. HIV RT-.

Two different pre-pro- leader sequence of mating factor alpha has been utilized to direct the expression and secretion of HIV RT proteins in yeast. The first one is pre-pro-MFalpha sequence of alpha mating factor of SEQ ID NO: 1 (PRE-PRO-MFalpha-RT) and the second one is pre-pro sequence of alpha mating factor deleted of the MFalpha sequence of SEQ ID NO: 2(PRE-PRO-RT).

The RT activity was assayed for the two fusion constructs and compared to the RT activity resulting from the RT coding sequence not fused to any signal peptide (No RT). As shown in figure 8, RT activity is five to six times higher for the two fusion constructs. This is clearly indicative of a higher level of HIV RT secretion in the medium of the transformed yeast cell.

### 2 - HIV RT assay on HIV-1 infected patients

Reverse Transcriptase sensitivity to inhibitors is measured for a reference and three patients infected by HIV-1 as described in Example 8.

Reverse Transcriptase inhibition is calculated as % of Reverse Transcriptase activity in the presence of each inhibitor concentration compared to Reverse Transcriptase activity in the absence of any inhibitor.

The Reverse Transcriptase reference comes from the isolate HIV-1 IIIB.

IC50 values are defined as the inhibitor concentrations for which Reverse Transcriptase activity is reduced by half.

The ratio of the IC50 value obtained for a Reverse Transcriptase from an infecting virus over the IC50 value of the Reverse Transcriptase reference virus for each inhibitor shows the sensitivity or loss of sensitivity of an infecting virus to the tested drug inhibiting the viral Reverse Transcriptase activity.

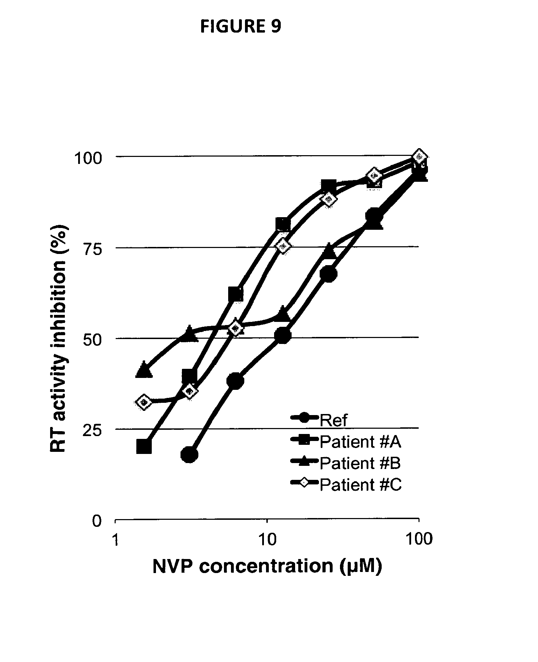

Increasing concentrations of the NNRTI inhibitor Nevirapine (NPV) were tested. Results are shown in figure 9 and in table below:

| **Name** | **Inhibitor** | **IC-50** | **R-Square** | **IC50 Ratio** |
|---|---|---|---|---|
| Ref | NVP | 12.11 | 1.00 | 1.00 |
| Patient #A | NVP | 4.29 | 0.94 | 0.35 |
| Patient #B | NVP | 4.45 | 0.99 | 0.37 |
| Patient #C | NVP | 6.13 | 0.98 | 0.51 |

The RT issued from the three patients #A, #B and #C are more sensitive to NVP inhibitor than the reference isolate HIV-1 IIIB.

Consequently, the three patients #A, #B and #C are infected by HIV virus sensitive to NVP inhibition and can be efficiently treated by such inhibitor.

## Claims

1. A method for identifying a compound inhibiting HIV reverse transcriptase (HIV RT), said method comprising the steps of :
a) Transforming a yeast cell by at least one nucleic acid encoding for a HIV RT and a signal peptide, wherein the nucleic acid sequence encoding for HIV RT is under the control of a promoter and the signal peptide enables the secretion of the HIV RT in the supernatant;
b) Culturing the transformed yeast cell of step a) for secretion of the HIV RT in the supernatant;
c) Separating the cultured transformed yeast cells of step b) from the supernatant for collecting said supernatant containing the HIV RT;
d) Assaying the activity of said HIV RT in the collected supernatant of step c) in the presence or absence of a compound; and
e) Selecting a compound inhibiting HIV RT activity in step d).

2. The method according to claim 1, wherein the at least one nucleic acid encoding for HIV RT is related to type 1 or type 2 HIV.

3. The method according to claim 2, wherein nucleic acid encoding for a HIV RT is obtained from a subject sample, preferably a human patient infected by HIV-1 or HIV-2.

4. The method according to claim 3, wherein the at least one nucleic acid encoding for HIV RT is amplified with a primer pair of:
- SEQ ID NO: 6 and SEQ ID NO: 7, or
- SEQ ID NO: 29 and SEQ ID NO: 5.

5. The method according to claim 4 wherein the at least one nucleic acid encoding for HIV RT is selected from the group comprising or consisting of SEQ ID NO: 12 and SEQ ID NO: 8.

6. The method according to claim 5, wherein the culture medium of the transformed yeast cell is deficient in tryptophan.

7. The method according to claim 1, wherein said method is for further identifying a compound inhibiting HIV protease (HIV PR), wherein:
- the step a) further comprises the transforming of said yeast cell by at least one nucleic acid encoding for a HIV PR, wherein the nucleic acid sequence encoding for HIV PR is under the control of a promoter;
- the step b) is for obtaining enough transformed yeast cells for testing HIV PR activity, and
- the step c) of separating the cultured transformed yeast cells from the supernatant is also for harvesting the cultured transformed yeast cell of step b); and
wherein said method further comprises the steps:
f) Assaying the activity of said HIV PR in the cultured transformed yeast cells in the presence or absence of a compound; and
g) Selecting a compound inhibiting HIV PR activity in step f).

8. The method according to claim 7, wherein the at least one nucleic acid encoding for HIV PR is amplified with a primer pair of:
- SEQ ID NO: 9 and SEQ ID NO: 10, or
- SEQ ID NO: 9 and SEQ ID NO: 11.

9. The method according to claim 8, wherein the at least one nucleic acid encoding for HIV PR is selected from the group comprising or consisting of SEQ ID NO: 12 and SEQ ID NO: 13.

10. The method according to claim 9, wherein the culture medium of the transformed yeast cell is also deficient in histidine.

11. A kit for identifying a compound inhibiting HIV RT, wherein said kit comprises:
(i) A yeast cell suitable for transformation,
(ii) A set of primers, and a vector, so as to obtain a nucleic acid encoding for a HIV RT and a signal peptide useful in the method for identifying a compound inhibiting HIV RT as defined in claims 1 to 10.

12. A kit according to claim 11, said kit further comprises:
(iii) A set of primers, and a vector, so as to obtain a nucleic acid encoding for a HIV PR useful in the method for identifying a compound inhibiting HIV PR as defined in claims 7 to 10,
wherein the sets of primers in (ii) and (iii) being identical or distinct.

13. Use of a kit as defined in claims 11 and 12 for identifying a compound inhibiting HIV RT.
